# EUROPEAN PATENT APPLICATION

(11) **EP 4 755 359 A1**
(43) Date of publication of application: **10.06.2026**
(21) Application number: 24849215.9
(22) Date of filing: 31.07.2024
(51) Int. Cl.: A61F 13/53, A61F 13/532

(54) **ABSORBENT ARTICLE**

(30) Priority: 01.08.2023 JP 2023125805
(71) Applicant: Daio Paper Corporation, Ehime 799-0492 (JP)
(72) Inventor: MORITANI, Akie, Shikokuchuo-shi, Ehime 799-0113 (JP)
(74) Representative: Meissner Bolte Partnerschaft mbB
(86) International application number: PCT/JP2024/027370
(87) International publication number: WO 2025/028570

(57) **Abstract**

[Problem] To improve retainability of super-absorbent polymer particles in a hardwood pulp fiber-containing absorbent body.

[Solution] The problem described above can be solved by an absorbent article including an absorbent element 50 having an absorbent body 56 and a wrapping sheet 58 that wraps the absorbent body 56, wherein the absorbent body 56 is made of mixing and aggregating pulp fibers and super-absorbent polymer particles, a percentage of particles of which a pre-swelling particle diameter is smaller than an average fiber length of hardwood pulp fibers in super-absorbent polymer particles 56p is 60% by mass or more, the absorbent element 50 is provided with a plurality of spaced high-compression portions 51 that are compressed in a thickness direction TD so as to be recessed into the absorbent body 56 from a surface of the absorbent element 50, portions other than the high-compression portions 51 in an arrangement region of the plurality of high-compression portions 51 are non-high-compression portions 52 that are thicker and lower in density than the high-compression portions 51, a shortest distance from each of the plurality of high-compression portions 51 to the closest other high-compression portion 51 is 1 to 4 mm, and an area of each of the high-compression portions 51 is 2 to 200 mm².

## Description

### Technical Field

The present invention relates to an absorbent article such as a disposable diaper, a sanitary napkin, an incontinence pad, or a panty liner (a vaginal discharge sheet).

### Background Art

An absorbent body (also referred to as an absorbent core or the like) generally contains pulp fibers and super-absorbent polymer particles. While softwood pulp (NBKP) fiber (pulp fiber derived from softwoods) has been widely used as pulp fiber, in recent years, the use of hardwood pulp (LBKP) fiber (pulp fiber derived from hardwoods) has also been considered due to the relatively low cost of hardwood pulp fiber as compared to softwood pulp fiber and the like (for example, refer to PTL 1 and 2).

However, an absorbent body made by mixing and aggregating pulp fibers containing both hardwood pulp fibers and softwood pulp fibers with super-absorbent polymer particles have a problem in that retainability of super-absorbent polymer particles is lower (super-absorbent polymer particles more readily migrate) compared to absorbent bodies made by mixing and aggregating pulp fibers only constituted of softwood pulp fibers and super-absorbent polymer particles.

### Citation List

### Patent Literature

PTL 1: Japanese Patent Application Publication No. 2020-014646
PTL 2: Japanese Patent No. 7293479

### Summary of Invention

### Technical Problem

In consideration thereof, a primary object of the present invention is to improve retainability of super-absorbent polymer particles in an absorbent body containing hardwood pulp fibers.

### Solution to Problem

Aspects of an absorbent article that achieves the object described above will be described below.

### <First aspect>

An absorbent article having a crotch portion, including:
an absorbent element including an absorbent body provided in a range in a front-back direction including the crotch portion, and a wrapping sheet that wraps the absorbent body, wherein
the absorbent body is made of mixing and aggregating pulp fibers including hardwood pulp fibers and super-absorbent polymer particles,
a percentage of particles of which a pre-swelling particle diameter is smaller than an average fiber length of the hardwood pulp fibers in the super-absorbent polymer particles is 60% by mass or more,
the absorbent element is provided with a plurality of spaced high-compression portions that are compressed in a thickness direction so as to be recessed into the absorbent body from at least one of front and back surfaces of the absorbent element,
portions other than the high-compression portions in an arrangement region of the plurality of high-compression portions are non-high-compression portions that are thicker and lower in density than the high-compression portions,
a shortest distance from each of the plurality of high-compression portions to the closest other high-compression portion is 1 to 4 mm, and
an area of each of the high-compression portions is 2 to 200 mm².

### (Operational effect)

The present inventor found that, in an absorbent body containing hardwood pulp fibers, due to a fiber length of the hardwood pulp fibers being short, entanglement of the pulp fibers are less likely to be maintained and, as a result, super-absorbent polymer particles more readily migrate. Therefore, while a longer fiber length of hardwood pulp fibers is preferable in order to prevent the migration of super-absorbent polymer particles, hardwood pulp fibers are naturally derived and there are limits to adjusting fiber length and other characteristics. Reducing a compounded amount of hardwood pulp fibers is also undesirable because such a reduction leads to lower cost advantages.

In contrast, as will become apparent from experiment results to be described later, providing the high-compression portions having the area and intervals according to the present aspect enables the retainability of the super-absorbent polymer particles to be improved in an absorbent body containing hardwood pulp fibers. In other words, due to a reduction in the distance between pulp fibers in the high-compression portions, entanglement of the pulp fibers is strengthened, voids among the pulp fibers that serve as migration paths of the super-absorbent polymer particles decrease, contact portions of the pulp fibers with respect to the super-absorbent polymer particles increase, and the like, thereby inhibiting the migration of the super-absorbent polymer particles within the absorbent body and the migration of the super-absorbent polymer particles to the outside of the absorbent body.

### <Second aspect>

The absorbent article according to claim 1, wherein
an area rate of the high-compression portions in an arrangement region of the plurality of high-compression portions is 10 to 35%, and
a diameter of a largest inscribed circle inscribed in an outline of the non-high-compression portions is 30 mm or less.

### (Operational effect)

As in the present aspect, since arranging the high-compression portions particularly densely makes it difficult for the super-absorbent polymer particles to migrate in the high-compression portions as described above and the migration of the super-absorbent polymer particles in the non-high-compression portions is blocked by the high-compression portions, an uneven distribution of the super-absorbent polymer particles is also suppressed. Therefore, retainability of the super-absorbent polymer particles further improves.

In addition, even in the non-high-compression portions, due to the absorbent body deforming near a periphery of the high-compression portions as if pulled by a deformation of the high-compression portions, an advantage in that the absorbent body becomes thinner compared to when the high-compression portions are absent is produced.

From these perspectives, it is particularly preferable if the area rate of the high-compression portions is within the range described above and the diameter of the largest inscribed circle inscribed in the outline of the non-high-compression portions is within the range described above so that the high-compression portions are not sparsely arranged (so that the non-high-compression portions do not continue long in all directions).

Since a dense arrangement of high-compression portions was conventionally considered less preferable, adopting a dense arrangement of high-compression portions as in the present aspect is not in the scope of optimization that is usually performed.

### <Third aspect>

The absorbent article according to the second aspect, wherein
the high-compression portions have an outline with no inflection points or bending points, the diameter of the largest inscribed circle inscribed in the outline of the high-compression portions is 1 to 10 mm, and a circumferential length of the outline of the high-compression portions is 1 to 15 times a circumferential length of the largest inscribed circle inscribed in the outline of the high-compression portions.

### (Operational effect)

Dimensions and a shape of the high-compression portions can be determined as appropriate. However, since the high-compression portions are hard portions, the absorbent body as a whole may lack flexibility or make a user feel as though a foreign body is mixed into the absorbent body (foreign body sensation) if the dimensions of the high-compression portions are excessively large, the shape of the high-compression portions is excessively long in one direction, or the shape has an excessively intricate outline. Therefore, the dimensions and the shape of the high-compression portions are preferably within the ranges according to the present aspect.

### <Fourth aspect>

The absorbent article according to the second or third aspect, wherein
when a virtual grid formed by a first virtual straight line along a first direction repeatedly arranged at a first interval in a second direction inclined at 80° to 90° clockwise in a plan view with respect to the first virtual straight line and a second virtual straight line along the second direction repeatedly arranged at a second interval in the first direction is defined in the arrangement region of the plurality of high-compression portions, and smallest virtual quadrangles with intersections of the first virtual straight line and the second virtual straight line as vertices are defined,
the high-compression portions are constituted of first high-compression portions arranged at intersection positions of the first virtual straight line and the second virtual straight line, second high-compression portions respectively arranged between adjacent first high-compression portions on the first virtual straight line and between adjacent first high-compression portions on the second virtual straight line, and third high-compression portions arranged at intersection positions of diagonals of the virtual quadrangles,
the first high-compression portions have a circular shape centered on an intersection of the first virtual straight line and the second virtual straight line,
the second high-compression portions have an elliptical shape or a rounded rectangle shape with a center of gravity at a midpoint of each side and a major axis along each side of the virtual quadrangles, and
the third high-compression portions have an elliptical shape or a rounded rectangle shape with a center of gravity at the intersections of the diagonals and a major axis along a front-back direction of the virtual quadrangles or a circular shape centered on the intersections of the diagonals of the virtual quadrangles.

### (Operational effect)

While the shape and the arrangement pattern of the high-compression portions in the arrangement region of the high-compression portions may be defined as appropriate, since the pattern according to the present aspect enables migration of the super-absorbent polymer particles in the non-high-compression portions to be kept within a region enclosed by the high-compression portions, an uneven distribution of the super-absorbent polymer particles can be suppressed using fewer high-compression portions and, at the same time, produces both a portion where the non-high-compression portions are linearly continuous in the first direction and a portion where the non-high-compression portions are linearly continuous in the second direction, the absorbent body as a whole is readily deformed so as to conform to a body surface even if individual high-compression portions harden.

### <Fifth aspect>

The absorbent article according to the fourth aspect, wherein
the first high-compression portions have a diameter of 1 to 4 mm,
the second high-compression portions have a major axis length of 3 to 6 mm and a minor axis length equal to a diameter of the first high-compression portions,
the third high-compression portions have same dimensions and a same shape as the second high-compression portions with the exception of an orientation of the major axis being along the front-back direction, and
a minimum interval between adjacent first high-compression portions and second high-compression portions is 1 to 2 mm.

### (Operational effect)

When the high-compression portions are arrayed along the virtual grid described earlier, while the dimensions of each high-compression portion can be defined as appropriate, the dimensions are preferably within the ranges according to the present aspect since not only retainability of the super-absorbent polymer particles but also deformability of the absorbent body improve.

### <Sixth aspect>

The absorbent article according to the fourth or fifth aspect, wherein
the virtual grid has a diagonal grid shape of which the first virtual straight line is inclined at 40 to 50° clockwise in a plan view with respect to the front-back direction and the second virtual straight line is inclined at 40° to 50° counterclockwise in a plan view with respect to the front-back direction.

### (Operational effect)

When the high-compression portions are arrayed along the virtual grid as described earlier, the high-compression portions are preferably arrayed in a diagonal grid shape as in the present aspect due to superior deformability of the absorbent body when worn.

### <Seventh aspect>

The absorbent article according to any one of first to sixth aspects, wherein
a basis weight of the pulp fibers in the absorbent body is 100 to 500 g/m²,
a ratio by weight of pulp fibers to super-absorbent polymer particles in the absorbent body is 40:60 to 65:35,
a thickness of the non-high-compression portions is 3 to 13 mm, and
a thickness of the high-compression portions is 60 to 90% of the thickness of the non-high-compression portions.

### (Operational effect)

In general, when improving a reversion prevention property, a mixing ratio of super-absorbent polymer particles with high liquid retention is often increased. In addition, in recent years, the mixing ratio of super-absorbent polymer particles is often increased for purposes such as making the absorbent body thinner. However, increasing the mixing ratio of super-absorbent polymer particles makes retainability of the super-absorbent polymer particles more likely to decrease. Therefore, compression and formation of the high-compression portions are preferably performed within the ranges according to the present aspect, with a composition of the pulp fibers and the super-absorbent polymer particles in the absorbent body within the ranges according to the present aspect.

### <Eighth aspect>

The absorbent article according to any one of first to seventh aspects, wherein
a percentage of particles of which a pre-swelling particle diameter is 500 µm or less in the super-absorbent polymer particles is 60% by mass or more, and
in all pulp fibers of the absorbent body, a percentage of pulp fibers with a fiber length of 0.5 mm or more and less than 1.1 mm is 40% or more by mass, and a percentage of pulp fibers with a fiber width of 10 µm or more and less than 35 µm is 90% or more by mass.

### (Operational effect)

Combining hardwood pulp fibers with fibers of longer average fiber length to realize a fiber length distribution of the pulp fibers according to the present aspect enables liquid retention capacity of the fibers due to capillary action to be provided while ensuring an absorption capacity and an absorption speed of the absorbent body, thereby improving the reversion prevention property. However, this by itself leaves room for improvement in terms of retainability of super-absorbent polymer particles. Therefore, preferably, the high-compression portions described earlier are adopted in combination to also improve retainability of super-absorbent polymer particles.

### <Ninth aspect>

The absorbent article according to the eighth aspect, wherein
the pulp fibers of the absorbent body are constituted of softwood pulp fibers and the hardwood pulp fibers, and
a mass ratio of the hardwood pulp fibers to the softwood pulp fibers is 25/75 or higher and 38/62 or lower.

### (Operational effect)

As will be made apparent in the experimental examples described later, the composition of the pulp fibers of the absorbent body according to the present aspect is particularly preferable.

### Advantageous Effects of Invention

According to the present invention, in an absorbent body containing hardwood pulp fibers, advantages such as an improvement in retainability of super-absorbent polymer particles can be achieved.

### Brief Description of Drawings

[Fig. 1]

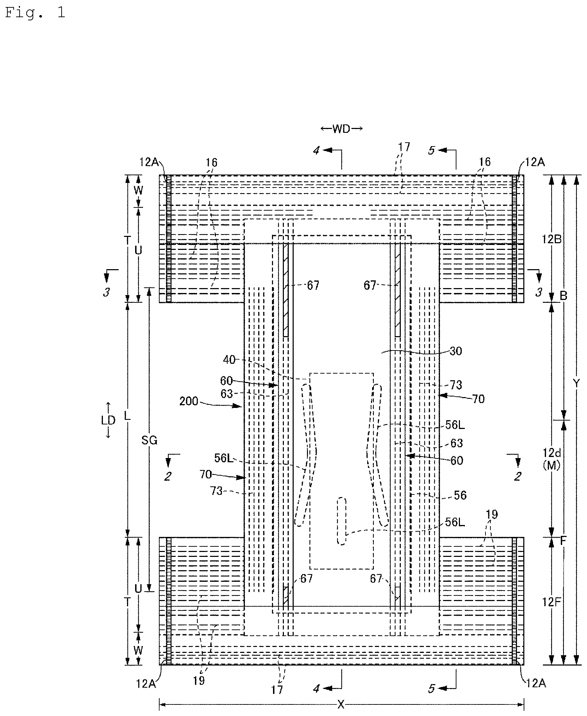

   Fig. 1 is a plan view showing an inner surface of a pant-type disposable diaper in an expanded state.
[Fig. 2]

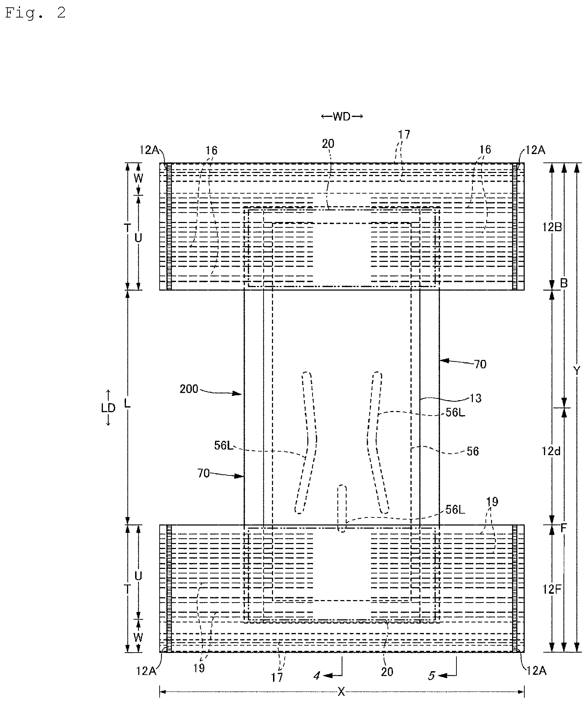

   Fig. 2 is a plan view showing an outer surface of a pant-type disposable diaper in an expanded state.
[Fig. 3]

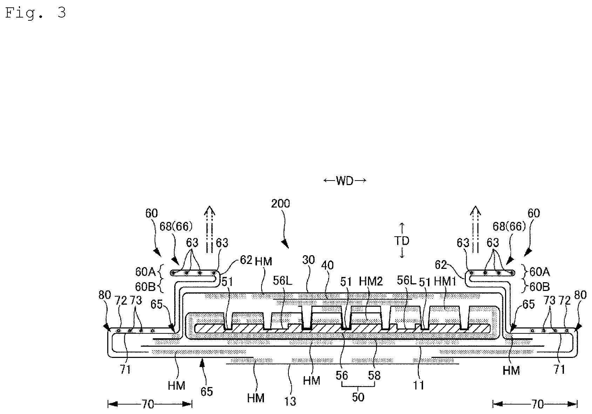

   Fig. 3 is a sectional view taken along 2-2 in Fig. 1.
[Fig. 4]

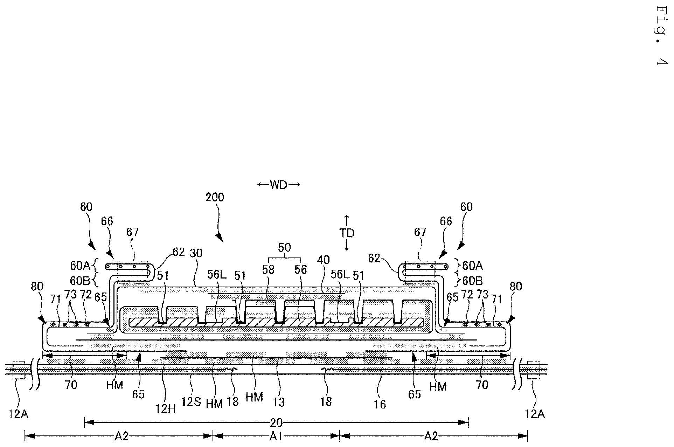

   Fig. 4 is a sectional view taken along 3-3 in Fig. 1.
[Fig. 5]

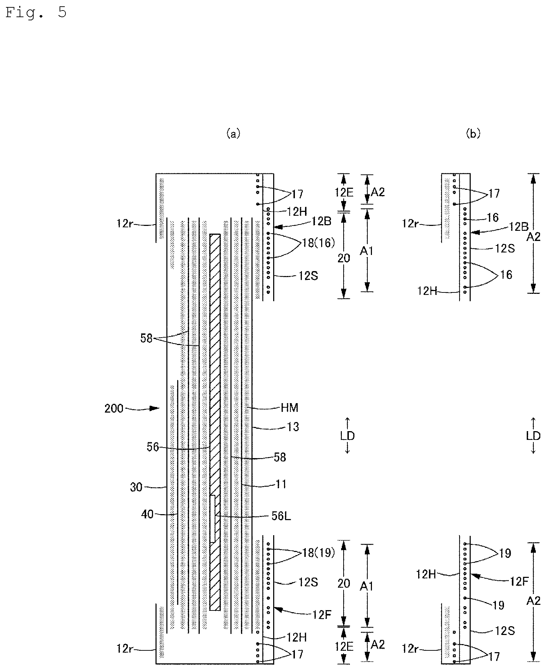

   Fig. 5(a) is a sectional view taken along 4-4 in Fig. 1 and Fig. 5(b) is a sectional view taken along 5-5 in Fig. 1.
[Fig. 6]

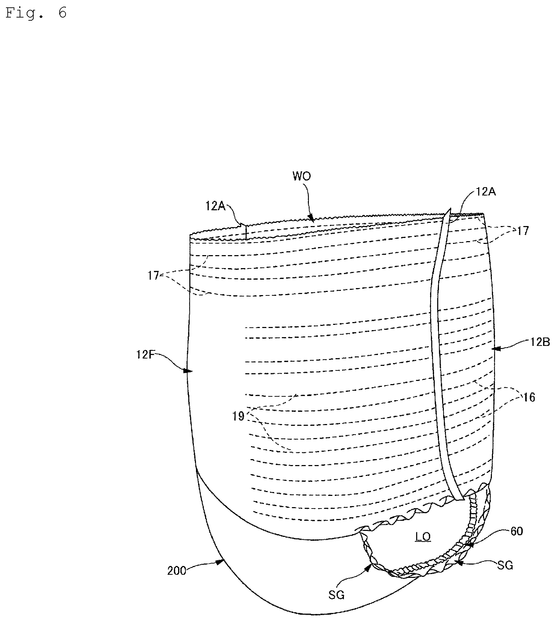

   Fig. 6 is a perspective view of a pant-type disposable diaper.
[Fig. 7]

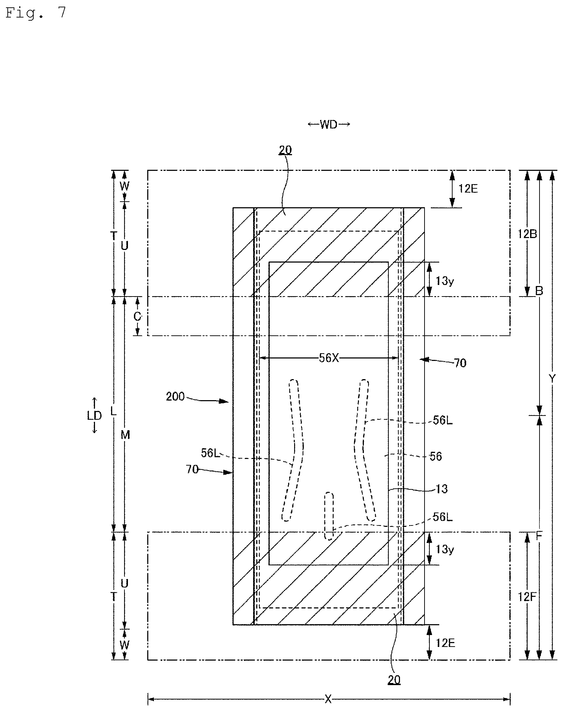

   Fig. 7 is a plan view showing an outer surface of an interior body in an expanded state together with a contour of an exterior body.
[Fig. 8]

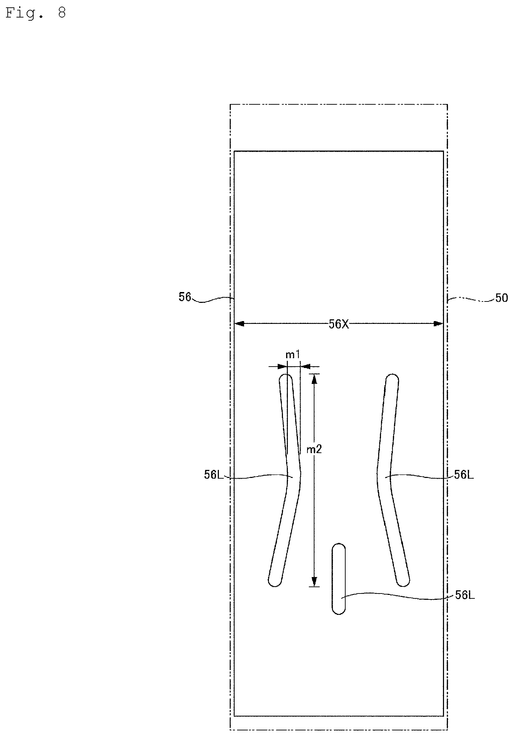

   Fig. 8 is a plan view showing a surface of an absorbent body together with a contour of a wrapping sheet.
[Fig. 9]

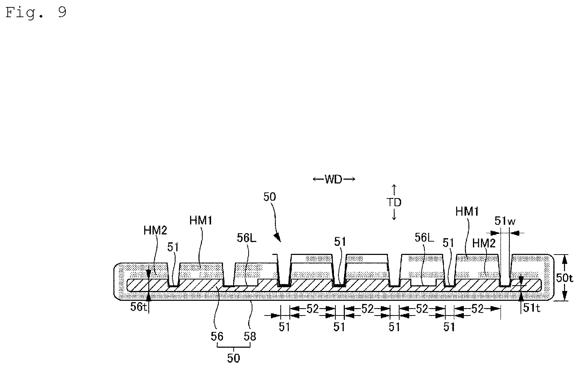

   Fig. 9 is a sectional view of an absorbent element.
[Fig. 10]

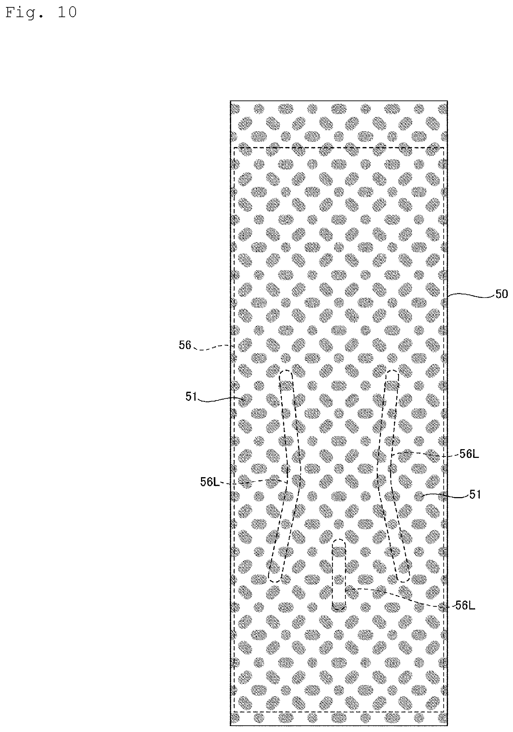

   Fig. 10 is a plan view of the absorbent element.
[Fig. 11]

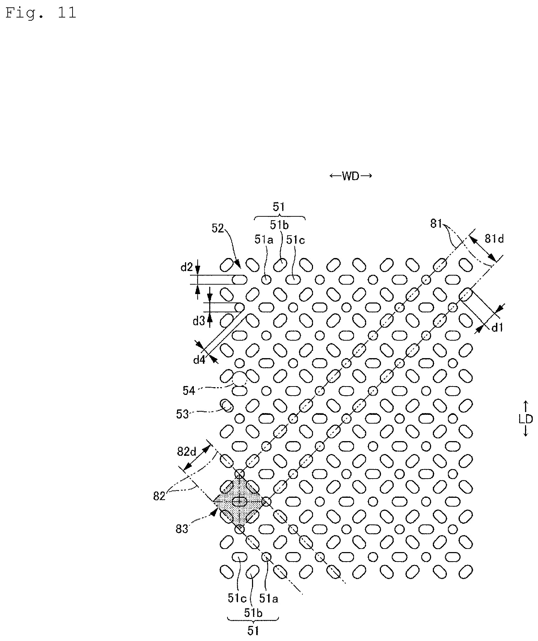

   Fig. 11 is a plan view showing enlarged details of a main part of a surface of the absorbent element.
[Fig. 12]

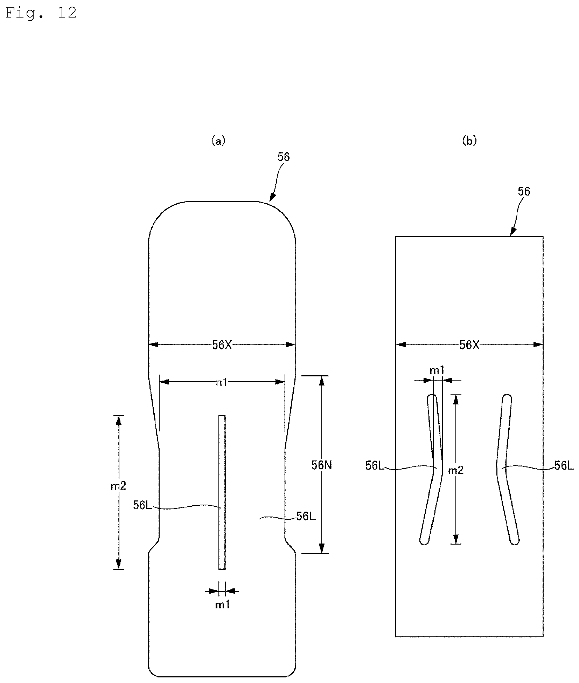

   Fig. 12 is a plan view showing another example of the absorbent body.
[Fig. 13]

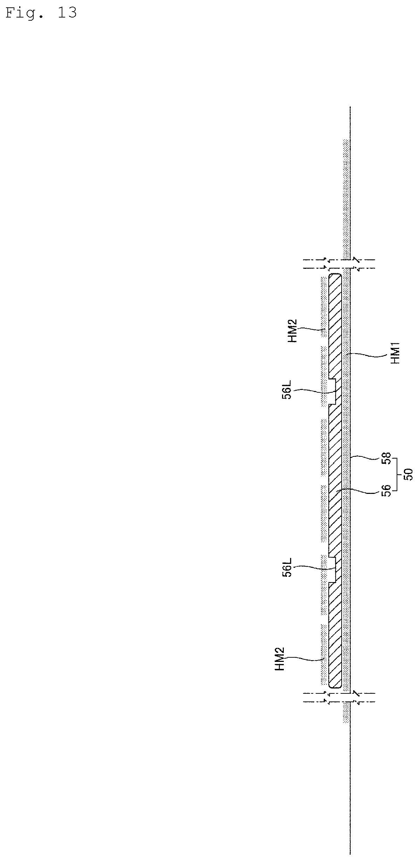

   Fig. 13 is a sectional view showing the absorbent element in a state prior to wrapping the absorbent body with the wrapping sheet.
[Fig. 14]

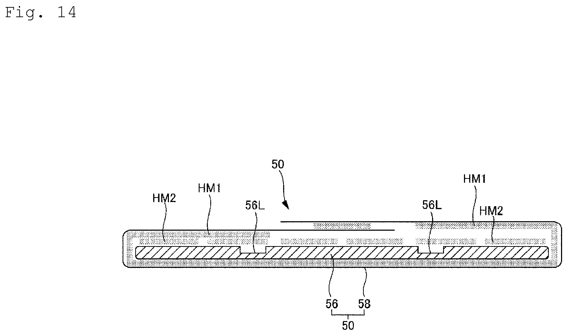

   Fig. 14 is a sectional view showing the absorbent element in a state prior to forming high-compression portions after wrapping the absorbent body with the wrapping sheet.
[Fig. 15]

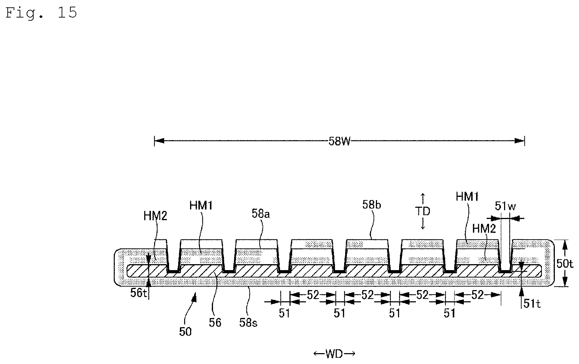

   Fig. 15 is a sectional view of another absorbent element.
[Fig. 16]

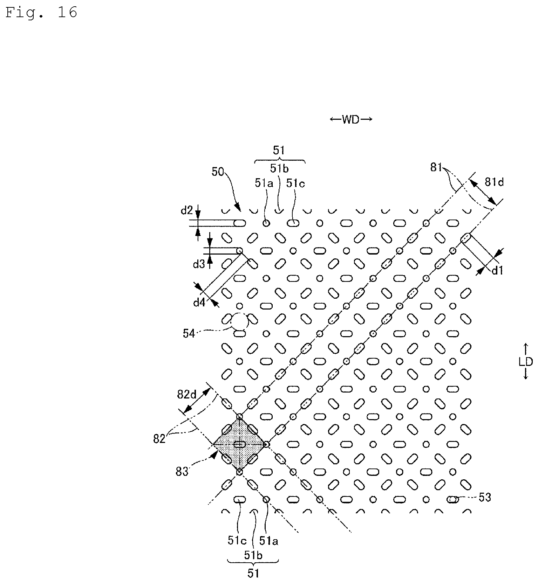

   Fig. 16 is a plan view showing enlarged details of a main part of a surface of the absorbent element.
[Fig. 17]

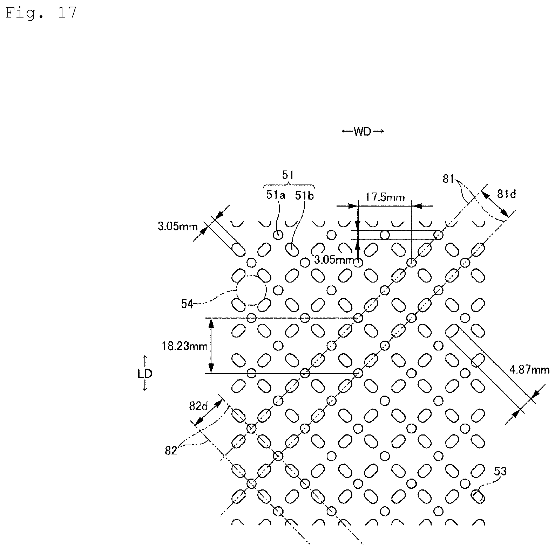

   Fig. 17 is a plan view showing enlarged details of a main part of a surface of the absorbent element.
[Fig. 18]

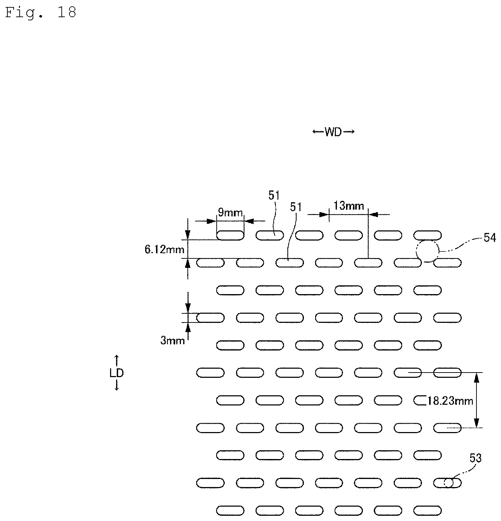

   Fig. 18 is a plan view showing enlarged details of a main part of a surface of the absorbent element.
[Fig. 19]

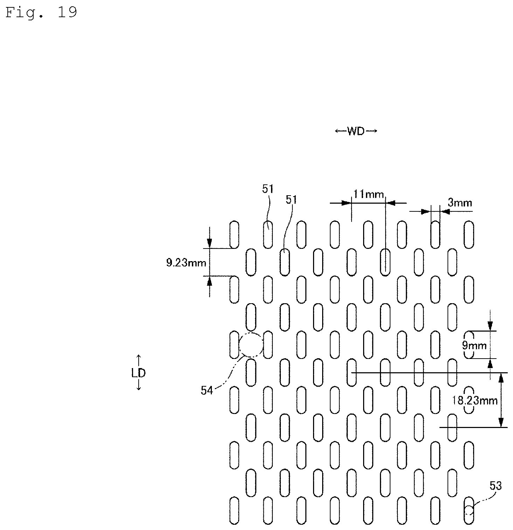

   Fig. 19 is a plan view showing enlarged details of a main part of a surface of the absorbent element.
[Fig. 20]

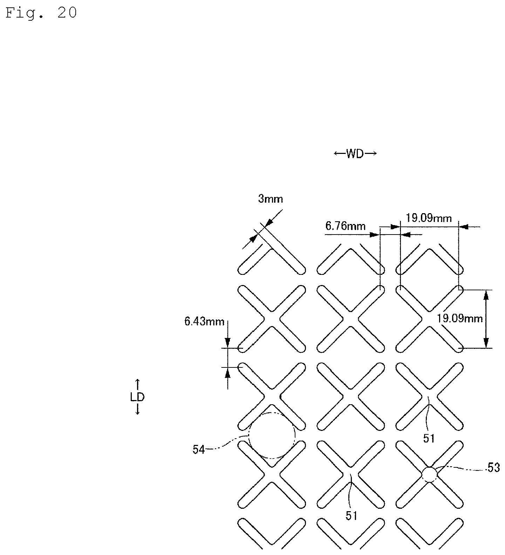

   Fig. 20 is a plan view showing enlarged details of a main part of a surface of the absorbent element.
[Fig. 21]

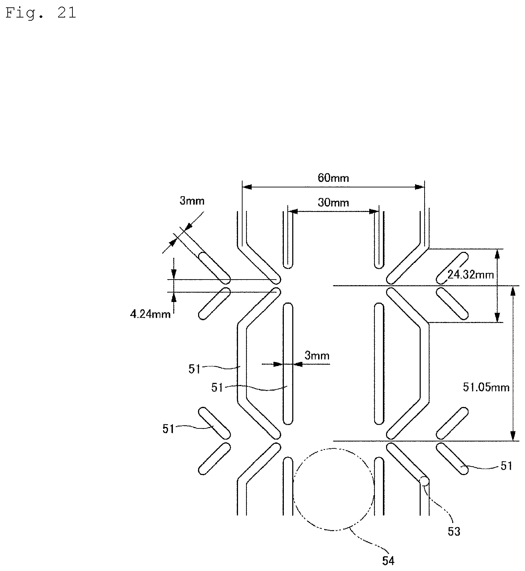

   Fig. 21 is a plan view showing enlarged details of a main part of a surface of the absorbent element.
[Fig. 22]

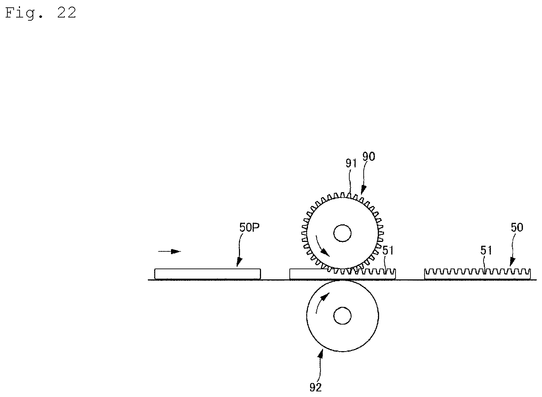

   Fig. 22 is a schematic view showing a manufacturing method of the absorbent element.
[Fig. 23]

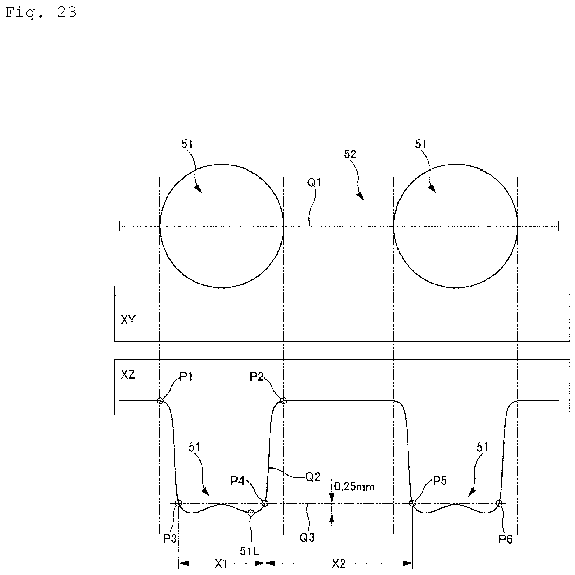

   Fig. 23 is an explanatory diagram of a depth measuring method of a high-compression portion.
[Fig. 24]

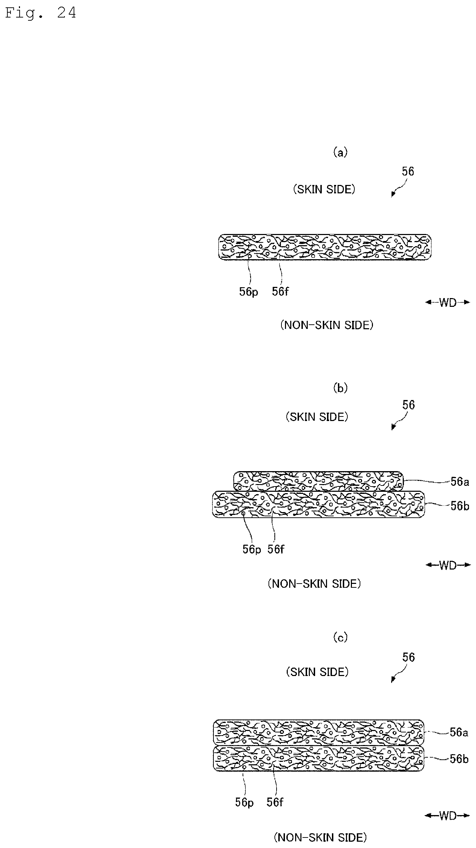

   Fig. 24 is a sectional view schematically showing various kinds of absorbent bodies.
[Fig. 25]

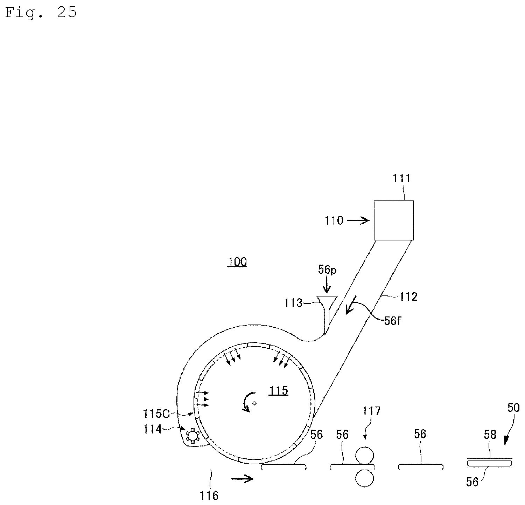

   Fig. 25 is a sectional view showing an example of a manufacturing facility of the absorbent element.
[Fig. 26]

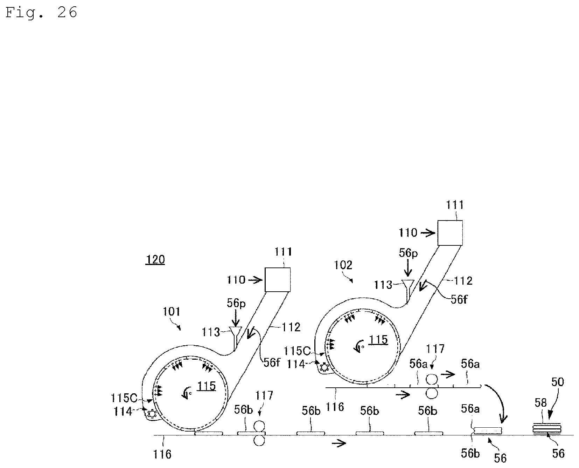

   Fig. 26 is a sectional view showing an example of a manufacturing facility of the absorbent element.
[Fig. 27]

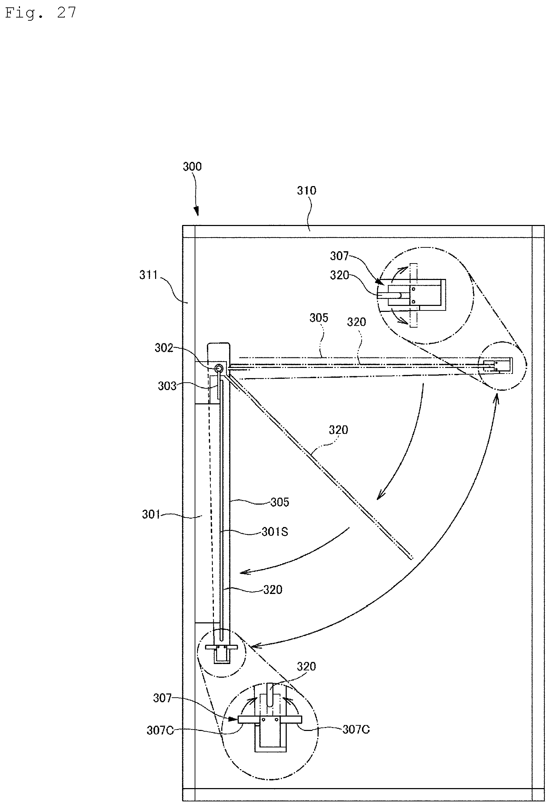

   Fig. 27 is a side view schematically showing an impact tester.
[Fig. 28]

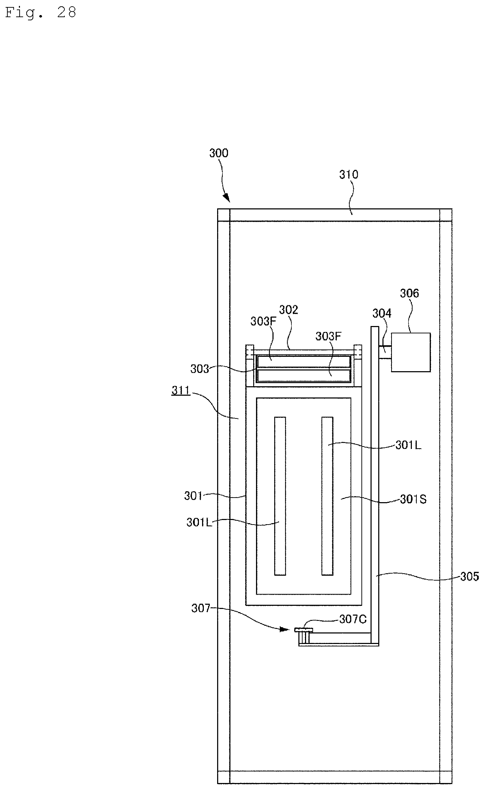

   Fig. 28 is a front view schematically showing the impact tester.

### Description of Embodiments

Hereinafter, a pant-type disposable diaper will be described in detail while referring to the accompanying drawings as an example of an absorbent article. In portions other than the fixed or joined portions described below, each component adjacent to each other in a thickness direction is fixed or joined as necessary in the same manner as in known diapers. Portions with dotted patterns in sectional views indicate an adhesive such as a hot-melt adhesive as means for such fixing or joining. Hot-melt adhesives can be applied by known methods such as slot application, continuous linear or dotted bead application, spray application in a spiral shape, a Z-shape, a wavy shape, or the like, or pattern coating (transfer of hot-melt adhesive by letterpress method). In place of or in addition to the above, in a fixing portion of an elastic member, a hot-melt adhesive may be applied to an outer peripheral surface of the elastic member and the elastic member may be fixed to an adjacent member. While examples of hot-melt adhesives include EVA-based, adhesive rubber-based (elastomer-based), polyolefin-based, and polyester/polyamide-based hot-melt adhesives, such hot-melt adhesives can be used without any particular limitations. As the fixing or joining means for joining each component, means that involve material welding such as heat sealing or ultrasonic sealing can be used. In portions where liquid permeability in a thickness direction is required, adjacent components in the thickness direction are fixed or joined in an intermittent pattern. For example, when such intermittent fixing or joining is to be performed using a hot-melt adhesive, intermittent pattern application in a spiral shape, a Z-shape, or a wavy shape can be suitably used, and when applying to an area that is equal to or larger than an application width by a single nozzle, intermittent pattern application in a spiral shape, a Z-shape, or a wavy shape can be performed with or without spacing in a width direction. As the joining means for joining each component, means that involve material welding such as heat sealing or ultrasonic sealing can also be used.

In addition, as nonwovens in the following description, known nonwovens can be used as appropriate depending on the site and purpose. As constituent fibers of nonwovens, synthetic fibers such as polyolefin-based synthetic fibers including polyethylene or polypropylene, polyesterbased synthetic fibers, and polyamide-based synthetic fibers (including composite fibers such as core-sheath fiber in addition to single component fibers), recycled fibers such as rayon and cupra, and natural fibers such as cotton can be selected without any particular limitations, and a mixture of these fibers can also be used. In order to increase the flexibility of nonwovens, the constituent fibers are preferably crimped fibers. In addition, the constituent fibers of the nonwovens may be hydrophilic fibers (including fibers made hydrophilic by a hydrophilic agent), hydrophobic fibers, or water-repellent fibers (including fibers made water-repellent by a water-repellent agent). Furthermore, while nonwovens are generally classified according to fiber lengths, sheet forming methods, fiber binding methods, and lamination structures into short-fiber nonwovens, long-fiber nonwovens, spunbonded nonwovens, meltblown nonwovens, spunlace nonwovens, thermal bonded (air-through) nonwovens, needlepunched nonwovens, point bonded nonwovens, laminated nonwovens (including SMS nonwovens and SMMS nonwovens with a meltblown layer sandwiched between spunbonded layers), and the like, any of these nonwovens can be used.

Figs. 1 to 6 show an example of a pant-type disposable diaper. The present pant-type disposable diaper includes a rectangular front exterior body 12F that constitutes a front-side lower torso portion and a rectangular back exterior body 12B that constitutes a back-side lower torso portion, and an interior body 200 provided inside the exterior bodies 12F and 12B so as to extend from the front exterior body 12F through a crotch portion M to the back exterior body 12B. A side seal 12A is formed by joining both side portions of the front exterior body 12F and both side portions of the back exterior body 12B and, accordingly, an opening formed by front and back end portions of the exterior bodies 12F and 12B are a waist opening WO through which a wearer's lower torso passes and portions respectively surrounded by lower edges of the exterior bodies 12F and 12B and a side edge of the interior body 200 on both sides in a width direction of the interior body 200 are leg openings LO through which the legs pass. The interior body 200 is a portion that absorbs and holds bodily exudates such as urine and the exterior bodies 12F and 12B are portions for supporting the interior body 200 with respect to the body of the wearer. In addition, reference sign Y denotes a total length of the diaper (a length in a front-back direction from an edge of the waist opening WO of a front body F to an edge of the waist opening WO of a back body B) in an expanded state and a reference sign X denotes a total width of the diaper in an expanded state.

The present pant-type disposable diaper has a lower torso region T that is defined as a front-back direction range having side seals 12A (front-back direction range from the waist opening WO to an upper end of the leg openings LO) and an intermediate region L that is defined as a front-back direction range of portions that form the leg openings LO (between a front-back direction region having the side seal 12A of the front body F and a front-back direction region having the side seal 12A of the back body B). Portions positioned in the lower torso region T in the front exterior body 12F and the back exterior body 12B or, in other words, a front-side lower torso portion and a back-side lower torso portion can be conceptually divided into a "waist portion" W that forms an edge portion of the waist opening and a "under-waist portion" U that is a portion below the "waist portion" W. Normally, when there is a boundary where a stretching stress in a width direction WD changes (for example, where a thickness or a stretch rate of the elastic member changes) within the front-side lower torso portion and the back-side lower torso portion, the waist opening WO side rather than the boundary nearest to the waist opening WO side becomes the waist portion W, and when such a boundary does not exist, a waist extension portion 12E that extends toward the waist opening WO side beyond the absorbent body 56 or the interior body 200 becomes the waist portion W. While lengths of these portions in the front-back direction differ depending on a size of the product and can be defined as appropriate, in one example, the waist portion W can range from 15 to 40 mm and the under-waist portion U can range from 65 to 120 mm. On the other hand, both side edges of the intermediate region L are constricted in a C-shape or a curved shape so as to conform to the circumferences of the legs of the wearer and constitute sites where the wearer inserts his/her legs. As a result, the pant-type disposable diaper in an expanded state approximately resembles an hourglass shape as a whole.

### (Exterior body)

As in the illustrated example, the exterior bodies 12F and 12B are constituted of the rectangular front exterior body 12F that is a portion constituting at least the lower torso portion of the front body F and the rectangular back exterior body 12B that is a portion constituting at least the lower torso portion of the back body B, and the front exterior body 12F and the back exterior body 12B are not continuous on the crotch side and may be separated in the front-back direction LD (two-piece exterior body type) or, although not illustrated, may be continuous from the front body to the back body (integrated exterior body type). A separation distance 12d in the front-back direction in the two-piece exterior body type can be set to, for example, approximately 40% to 60% of a total length Y. While lower edges of the front exterior body 12F and the back exterior body 12B have a linear shape along the width direction WD in the illustrated example, at least a part of the lower edges of the front exterior body 12F and the back exterior body 12B may have a curved shape that conforms to the circumferences of the legs.

In a pant-type disposable diaper of the two-piece exterior body type, since the interior body 200 is exposed between the front exterior body 12F and the back exterior body 12B, a back surface of the interior body 200 is preferably equipped with a cover nonwoven 13 extending from between the front exterior body 12F and the interior body 200 to between the back exterior body 12B and the interior body 200 so as to prevent a liquid-impermeable sheet 11 from being exposed on the back surface of the interior body 200. An inner surface and an outer surface of the cover nonwoven 13 can be respectively bonded to opposing surfaces via a hot-melt adhesive. The nonwoven used for the cover nonwoven 13 can be appropriately selected from, for example, materials similar to those used for the exterior bodies 12F and 12B. Although not illustrated, the exterior body may be continuous through the crotch from the front body F to the back body B. In this case, the exterior body not only has a portion corresponding to the lower torso region T but also has a portion corresponding to the intermediate region L.

The front exterior body 12F and the back exterior body 12B have a front lower torso portion and a back-side lower torso portion that constitute the lower torso region T. While dimensions of the front exterior body 12F and the back exterior body 12B in the front-back direction LD are equal to each other and the front exterior body 12F and the back exterior body 12B do not have portions corresponding to the intermediate region L in the example shown in Figs. 1 and 2, as shown in Fig. 7, the dimension in the front-back direction of the back exterior body 12B may be longer than that of the front exterior body 12F, and while the front exterior body 12F does not have a portion corresponding to the intermediate region L, the back exterior body 12B may have a gluteal cover portion C that extends out to the side of the intermediate region L from the lower torso region T. Although not illustrated, the front exterior body 12F may also be provided with a groin cover portion that extends out to the side of the intermediate region L from the lower torso region T.

As shown in Figs. 4 and 5, in the exterior bodies 12F and 12B, outside sheet layers and inside sheet layers respectively adjacent to insides and outsides of elastic members 16 to 19 (to be described later) are joined by joining means such as a hot-melt adhesive or welding. In addition to being formed by two sheet materials 12S and 12H as in the illustrated example, the inside sheet layers and the outside sheet layers can also be formed by one sheet material. For example, in the case of the latter, each of the inside sheet layers and the outside sheet layers is formed in a part or all of the exterior bodies 12F and 12B by an inside portion and an outside portion of one sheet material folded back at the edge of the waist opening WO (or an edge on a crotch side). The illustrated example is an example of the former in which the sheet material 12S forming the outside sheet layer in the under-waist portion is folded back to the inside of the sheet material 12H forming the inside sheet layer in the under-waist portion around the side of the waist opening WO of the sheet material 12H, and a folded-back portion 12r extends so as to cover up to an end portion on the side of the waist opening WO of the interior body 200. On the other hand, in the waist portion, the folded-back portion 12r constitutes the inner sheet layer adjacent to the inside of the elastic member.

The elastic members 16 to 19 are built into the exterior bodies 12F and 12B in order to enhance fit around the lower torso of the wearer and a stretchable region A2 that elastically stretches in the width direction WD with the stretching of the elastic members 16 to 19 is formed. In the stretchable region A2, the exterior bodies 12F and 12B contract in a natural-length state as the elastic member contracts, wrinkles or folds are formed, and when stretching in a longitudinal direction of the elastic member, the exterior bodies 12F and 12B can stretch up to a predetermined stretch rate at which the exterior bodies 12F and 12B fully stretch without any wrinkles. As the elastic members 16 to 19, in addition to elastic members with an elongated shape such as thread rubber (illustrated example), known elastic members with a strip shape, a net shape, a film shape, or the like can be used without limitation. Both synthetic rubber and natural rubber may be used as the elastic members 16 to 19.

To explain the elastic members 16 to 19 in the illustrated example in greater detail, a plurality of waist elastic members 17 are attached at intervals in the front-back direction to the waist portions W of the exterior bodies 12F and 12B so as to be continuous across the entire width direction WD. In addition, one or a plurality of waist elastic members 17 arranged in a region adjacent to the under-waist portion U among the waist elastic members 17 may overlap with the interior body 200 or may be respectively provided on both sides in the width direction so as to avoid a central portion in the width direction that overlaps with the interior body 200. As the waist elastic member 17, around 2 to 15 pieces and, particularly, around 4 to 10 pieces of thread rubber with a thickness of 155 to 1880 dtex and, particularly, around 470 to 1240 dtex (in case of synthetic rubber) (in case of natural rubber, with a sectional area of 0.05 to 1.5 mm² and, particularly, around 0.1 to 1.0 mm²) are preferably arranged at intervals of 2 to 12 mm and, particularly, 3 to 7 mm, and a stretch rate in the width direction WD of the resulting waist portion W is preferably 150 to 400% and, particularly, around 220 to 320%. In addition, an elastic member with a same size need not be used or the stretch rate of the elastic member need not be the same over the entire waist portion W in the front-back direction LD and, for example, the thickness or the stretch rate may partially differ.

Furthermore, in the under-waist portion U of the exterior bodies 12F and 12B, a plurality of under-waist elastic members 16 and 19 constituted of elastic members with an elongated shape are attached at intervals in the front-back direction to form a under-waist stretchable region (region having the under-waist elastic members 16 and 19). As the under-waist elastic members 16 and 19, around 5 to 30 pieces of thread rubber with a thickness of 155 to 1880 dtex and, particularly, around 470 to 1240 dtex (in case of synthetic rubber) (in case of natural rubber, with a sectional area of 0.05 to 1.5 mm² and, particularly, around 0.1 to 1.0 mm²) is preferably arranged at intervals of 1 to 15 mm and, particularly, 3 to 8 mm, and a stretch rate in the width direction WD of the resulting under-waist portion U is preferably 200 to 350% and, particularly, around 240 to 300%. In addition, an elastic member with a same size need not be used or the stretch rate of the elastic member need not be the same over the entire under-waist portion U in the front-back direction LD and the thickness or the stretch rate may partially differ.

When the elastic members 16 and 19 are provided in a range in the front-back direction having the absorbent body 56 as in the under-waist portion U of the illustrated example, in order to prevent contraction of the absorbent body 56 in the width direction WD in a part of or all of the range, preferably, the middle in the width direction that includes a part of or all of a portion overlapping the absorbent body 56 in the width direction WD may be a non-stretchable region A1 and both sides of the non-stretchable region A1 in the width direction may be stretchable regions A2 (in the illustrated example, under-waist stretchable regions) as shown in Figs. 4, 5, 12, and the like. While a dimension in the width direction of the stretchable regions A2 provided on both sides in the width direction of the non-stretchable region A1 is approximately constant in the front-back direction LD as in the illustrated example, although not illustrated, the dimension in the width direction of the stretchable regions A2 can also vary in the front-back direction LD. In addition, while the dimension in the width direction WD of the stretchable regions A2 provided on both sides in the width direction WD of the non-stretchable region A1 is approximately the same in the front body F and the back body B, the dimension can also differ between the front body F and the back body B.

The stretchable regions A2 and the non-stretchable region A1 described above can be constructed by attaching the elastic members 16 to 17 and 19 between the inside sheet layer and the outside sheet layer, subsequently, subjecting the elastic members 16 and 19 to pressure and heat or finely cutting the elastic members 16 and 19 by cutting in one location or over approximately the entire middle portion in the width direction of the region to become the non-stretchable region A1, and eliminating elasticity in the non-stretchable region A1 while retaining elasticity in the stretchable regions A2. Note that an unnecessary elastic member 18 that does not substantially contribute to the formation of elasticity is to remain in the non-stretchable region A1.

While sheet materials can be used as the sheet materials 12S and 12H forming the inside sheet layer and the outside sheet layer without particular limitation, nonwovens are preferable. When using a nonwoven, a basis weight per sheet is preferably around 10 to 30 g/m².

The elastic members 16 to 19 can be fixed to the exterior bodies 12F and 12B by known methods. In addition, the inside sheet layer and the outside sheet layer can also be joined to each other by known methods. For example, in the portions of the exterior bodies 12F and 12B that have the elastic members 16 to 19, by applying a hot-melt adhesive HM only to outer peripheral surfaces of the elastic members 16 to 19 using application means such as a comb gun or a SureWrap nozzle and sandwiching the elastic members 16 to 19 between the inside sheet layer and the outside sheet layer, the elastic members 16 to 19 can be fixed to the inside sheet layer and the outside sheet layer and the inside sheet layer and the outside sheet layer can be fixed to each other using only the hot-melt adhesive HM applied to the outer peripheral surfaces of the elastic members 16 to 19.

### (Interior body joint)

Joining of the interior body 200 to the exterior bodies 12F and 12B can be performed by joining means using material welding such as heat sealing or ultrasonic sealing or by a hot-melt adhesive. In the illustrated example, the interior body 200 is fixed to inner surfaces of the exterior bodies 12F and 12B via a hot-melt adhesive applied to the back surface of the interior body 200 or, in other words, a back surface of the liquid-impermeable sheet 11 in this case and a root portion 65 of rising gathers 60. An interior body joint 20 that joins the interior body 200 and the exterior bodies 12F and 12B to each other may be, as shown in Fig. 2, provided over approximately the entire region in which the interior body 200 and the exterior bodies 12F and 12B overlap with each other or may be provided in, for example, a portion excluding both end portions of the interior body 200 in the width direction.

### (Interior body)

While the interior body 200 can take any shape, the interior body 200 is a rectangle with long sides in the front-back direction LD in the illustrated example. As shown in Figs. 3 to 5, the interior body 200 includes a top sheet 30 to be a body side, the liquid-impermeable sheet 11, and an absorbent element 50 interposed therebetween. Reference sign 40 denotes an intermediate sheet (second sheet) provided between the top sheet 30 and the absorbent element 50 to quickly transfer liquid that has passed through the top sheet 30 to the absorbent element 50, and reference sign 60 denotes rising gathers 60 that extend out from both side portions of the interior body 200 so as to come into contact with the circumference of the legs of the wearer in order to prevent bodily exudates from leaking to both sides of the interior body 200.

### (Top sheet)

The top sheet 30 has liquid permeability and examples include a porous or non-porous nonwoven and a porous plastic sheet. In addition, the top sheet 30 may be constituted of a single sheet or a laminated sheet obtained by bonding together two or more sheets. In a similar manner, regarding a planar direction, the top sheet 30 may be constituted of a single sheet or two or more sheets.

Both side portions of the top sheet 30 may be folded back toward the back side at side edges of the absorbent element 50 or may protrude toward the side from the side edges of the absorbent element 50 without being folded back.

For the purpose of preventing misalignment with a member on a back side, the top sheet 30 is desirably fixed to the member adjacent on the back side by joining means using material welding such as heat sealing or ultrasonic sealing or by a hot-melt adhesive. In the illustrated example, the top sheet 30 is fixed to a surface of the intermediate sheet 40 and a surface of a portion positioned on a front side of the absorbent body 56 in a wrapping sheet 58 by the hot-melt adhesive applied to the back surface of the top sheet 30.

### (Intermediate sheet)

In order to quickly transfer liquid that has passed through the top sheet 30 to the absorbent body 56, the intermediate sheet (also referred to as a second sheet) 40 with a higher permeability rate of liquid than the top sheet 30 can be provided. The intermediate sheet 40 is designed to quickly transfer liquid to the absorbent body 56 to enhance absorption performance by the absorbent body 56 and to reduce reversion from the absorbent body 56. The intermediate sheet 40 can also be omitted.

Examples of the intermediate sheet 40 include materials similar to the top sheet 30, spunlace nonwovens, spunbonded nonwovens, SMS nonwovens, pulp nonwovens, mixed sheets of pulp and rayon, point-bonded nonwovens, and crepe paper. In particular, air-through nonwovens are preferable due to their bulkiness. Composite fibers with a core-sheath structure are preferably used as the air-through nonwovens and, in this case, while resin used as the core may be polypropylene (PP), polyester (PET) with high rigidity is preferable. A basis weight is preferably 17 to 80 g/m² and more preferably 25 to 60 g/m². A thickness of the raw material fibers of the nonwovens is preferably 2.0 to 10 dtex. In order to increase the bulkiness of the nonwovens, it is also preferable to use fibers with eccentric cores in which the cores are not centered, hollow fibers, or hollow fibers with eccentric cores for all of the raw material fibers or a part of mixed fibers that constitute the raw material fibers.

While the intermediate sheet 40 in the illustrated example is shorter than a width of the absorbent body 56 and arranged at center, the intermediate sheet 40 may be provided across the entire width. A length of the intermediate sheet 40 in the front-back direction may be the same as the entire length of the diaper, the same as the length of the absorbent element 50, or within a short length range centered on a region where liquid is received.

For the purpose of preventing misalignment with a member on a back side, the intermediate sheet 40 is desirably fixed to the member adjacent on the back side by joining means using material welding such as heat sealing or ultrasonic sealing or by a hot-melt adhesive. In the illustrated example, the intermediate sheet 40 is fixed to a surface of a portion positioned on a front side of the absorbent body 56 in the wrapping sheet 58 by the hot-melt adhesive applied to the back surface of the intermediate sheet 40.

### (Liquid-impermeable sheet)

Although the material of the liquid-impermeable sheet 11 is not particularly limited, examples include plastic films constituted of polyolefin-based resins such as polyethylene and polypropylene, laminated nonwovens in which a plastic film is provided on surfaces of the nonwovens, and laminated sheets obtained by laminating and bonding nonwovens or the like to plastic films. Preferably, a liquid-impermeable and moisturepermeable material preferably used from the perspective of preventing humidity buildup is used as the liquid-impermeable sheet 11. As a plastic film with moisture permeability, microporous plastic films obtained by kneading inorganic fillers into polyolefin-based resins such as polyethylene and polypropylene, molding sheets, and then stretching the molded sheets in one or two axial directions are widely used. In addition to the above, while nonwovens made from microdenier fibers, nonwovens with enhanced leak resistance by reducing voids between fibers by applying heat or pressure, sheets that are made liquid-impermeable without using plastic films by methods such as coating with high water-absorption resin or hydrophobic resin or water repellent agents can also be used as the liquid-impermeable sheet 11, resin films are desirably used in order to obtain sufficient adhesive strength when bonding with the cover nonwoven 13 (to be described later) using a hot-melt adhesive.

In addition to setting a width of the liquid-impermeable sheet 11 so that the liquid-impermeable sheet 11 fits on a back side of the absorbent element 50 as illustrated, the liquid-impermeable sheet 11 can also be wrapped around both sides of the absorbent element 50 and extended to both side portions of a surface of the absorbent element 50 on the side of the top sheet 30. A suitable width of this extended portion is approximately 5 to 20 mm on each of left and right sides.

### (Absorbent element)

The absorbent element 50 has the absorbent body 56 and the wrapping sheet 58 that completely envelops the absorbent body 56. The absorbent element 50 has the crotch portion M and portions that extend to a front side and a back side of the crotch portion M.

### (Absorbent body)

The absorbent body 56 need only extend to both front and back sides of the crotch portion M so as to include the crotch portion M. In the case of a pant-type disposable diaper such as that according to the present example, the absorbent body 56 preferably extends to a peripheral edge portion of the interior body 200 or a vicinity thereof in the front-back direction LD and the width direction WD. Note that reference sign 56X denotes a full width of the absorbent body 56.

When making it easier to ensure absorption capacity in the crotch portion M, the absorbent body 56 is preferably approximately rectangular in shape as in the example shown in Fig. 8. In addition, in order to improve fit in the crotch portion M as in the example shown in Fig. 12(a), the width of the absorbent body 56 in the crotch portion M may be made narrower than both front and back sides of the crotch portion M to form a constricted shape. In this case, in order to make it easier to ensure absorption capacity in the crotch portion M, a width n1 of a narrowest portion of the absorbent body 56 in the crotch portion M is preferably 0.85 times or more of the full width 56X of the absorbent body 56.

Note that when the absorbent body 56 has a narrowest portion 56n (to be described later), the crotch portion M refers to a range in the front-back direction LD that includes the narrowest portion 56n, when the absorbent body 56 does not have the narrowest portion 56n but an outline of the diaper in the expanded state has a narrowest portion as in the illustrated example, the crotch portion M refers to a range in the front-back direction LD that includes the narrowest portion of the outline of the diaper (in the illustrated example, between the front exterior body 12F and the back exterior body 12B), and when the absorbent body 56 has neither narrowest portion, the crotch portion M refers to a portion which is positioned at center in the front-back direction LD and of which a dimension in the front-back direction LD is 20 to 30% of a total length of the product. Portions respectively extending toward the front side and the back side from the crotch portion M constitute the front-side portion and the back-side portion.

The absorbent body 56 may be a single layer as shown in Figs. 3 and 24(a) or a stack of a plurality of layers as shown in Figs. 24(b) and 24(c). A layer of the absorbent body 56 is obtained through a fiber-stacking process by an integrated model of a predetermined fiber-stacking drum and, if necessary, a compression process in the thickness direction. Therefore, for example, as shown in Figs. 24(b) and 24(c), the absorbent body 56 may be constituted of two layers which are an upper layer 56a and a lower layer 56b.

Note that sizes of the plurality of layers included in the absorbent body 56 may be the same or may differ from each other. In addition, thicknesses of the plurality of layers included in the absorbent body 56 may also be the same or may differ from each other. For example, widths and thicknesses of the upper layer 56a and the lower layer 56b included in the absorbent body 56 may differ from each other as shown in Fig. 24(a). In the example shown in Fig. 24(b), the width and the thickness of the upper layer 56a are smaller than those of the lower layer 56b. The sizes of the plurality of layers included in the absorbent body can be set according to the form and intended use of the absorbent article. Note that the number of layers is not limited to two and may be three or more.

A thickness of the absorbent body 56 (when the absorbent body is constituted of a plurality of layers, a total thickness of the plurality of layers) can be 0.5 to 30 mm and, preferably, 1 to 15 mm. For example, in the case of a disposable diaper, the thickness of the absorbent body 56 can be 1 to 30 mm, preferably 1.5 to 15 mm, and more preferably 5 to 13 mm. In addition, in the case of a sanitary napkin, the thickness of the absorbent body 56 can be 0.3 to 30 mm and preferably 1.0 to 15 mm. The thickness of the absorbent body 56 may be uniformed or regions of which the thickness is relatively thick or relatively thin may be formed.

The absorbent body 56 is made of mixing and aggregating pulp fibers 56f and super-absorbent polymer particles 56p, the pulp fibers 56f and the super-absorbent polymer particles 56p are present approximately uniformly throughout the entire absorbent body 56, and the super-absorbent polymer particles 56p are retained between the pulp fibers 56f. The absorbent body 56 described above can be manufactured using a fiber-stacking drum to be described later. While the absorbent body 56 is desirably made of mixing and aggregating only the pulp fibers 56f and the super-absorbent polymer particles 56p, if necessary, the absorbent body 56 may contain substances other than the pulp fibers 56f and the super-absorbent polymer particles 56p such as a filler, a pigment, a sizing agent, a coagulant, an oil resistance agent, a sulfate band, a yield improver, a water filterability improver, a dry paper strength enhancer, a wet paper strength enhancer, a color pigment, a water proofing agent, a deodorant, a fragrance, and other additives.

### (Pulp fiber)

Pulp is an aggregate of fibers (pulp fibers) extracted from wood, grass, or other plants by a mechanical process and/or a chemical process. In addition to softwood and hardwood, bamboo, rice, kudzu, Japanese silver grass, hemp, sugarcane, and the like can also be used as a raw material of pulp. Furthermore, pulp made of artificial cellulose such as rayon and acetate can also be used.

While the pulp fibers 56f included in the absorbent body 56 preferably contains softwood pulp fibers (pulp fibers derived from softwoods) and pulp fibers such as hardwood pulp fibers (pulp fibers derived from hardwoods) of which an average fiber length is longer than that of softwood pulp fiber in a mixed state, the pulp fibers 56f may only contain softwood pulp fibers (100% softwood pulp fiber). Hereinafter, the absorbent body 56 described above will also be referred to as a hardwood pulp fiber-containing absorbent body. The hardwood pulp fibers are preferably bleached hardwood kraft pulp (LBKP) manufactured by the kraft method. In addition, the softwood pulp fibers are preferably bleached softwood kraft pulp (NBKP) manufactured by the kraft method.

A content of the softwood pulp fibers and the hardwood pulp fibers in all pulp fibers 56f is preferably 80% by mass or more, more preferably 90% by mass or more, even more preferably 95% by mass or more, and even more preferably 98% by mass or more. In addition, all pulp fibers 56f are preferably substantially constituted of softwood pulp fibers and hardwood pulp fibers. Note that in the present specification, unless stated otherwise, a content of the pulp fibers 56f or materials thereof refer to an absolute dry content or, in other words, a mass ratio in an absolute dry state.

As shown in Table 1, (I) in the case of the absorbent body 56 constituted of softwood pulp fibers, since a fiber length and a fiber width of the pulp fibers 56f are relatively large and variabilities of the fiber length and the fiber width are also relatively large, generally, distances between the pulp fibers 56f increase and the obtained absorbent body 56 is bulky. Therefore, an absorption capacity (water absorption) by the pulp fiber 56f is large and an absorption speed of the absorbent body 56 is relatively fast. However, due to the large distances between the pulp fibers 56f, a liquid retention ability of the fibers due to capillary action is relatively low, and furthermore, the super-absorbent polymer particles (SAP) tend to drop out in the direction of gravity, making retainability of the super-absorbent polymer particles (SAP) between the pulp fibers 56f somewhat inferior. As a result, compared to the absorbent bodies 56 of (II) and (III) below, the liquid retention ability as the absorbent body 56 decreases and an effect of preventing reversion of liquid is relatively low.

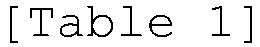

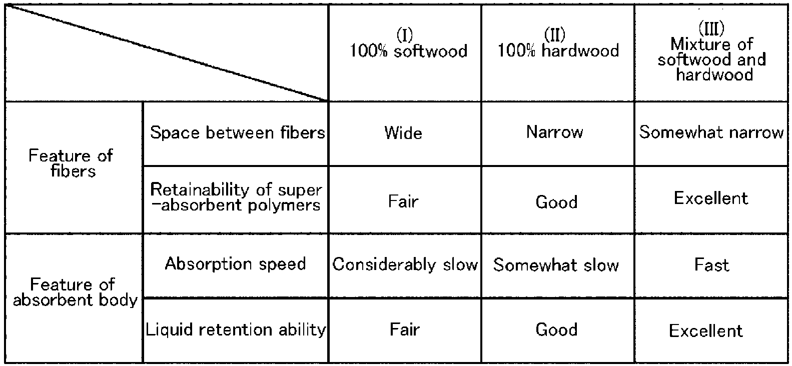

In addition, (II) in the absorbent body 56 constituted of hardwood pulp fibers, since the fiber length and the fiber width of the pulp fibers 56f are relatively small and variabilities of the fiber length and the fiber width are also relatively small, distances between the pulp fibers 56f decrease and fiber density of the obtained absorbent body 56 also increases. Therefore, the absorption capacity (water absorption) by the pulp fiber 56f is small and the absorption speed of the absorbent body 56 or an absorption speed as an absorbent article is slow. However, since the distance between the pulp fibers 56f is small, the liquid retention ability of the fibers due to capillary action is high. In addition, the retainability of the super-absorbent polymer particles 56p between the pulp fibers 56f is generally favorable and the liquid retention ability by the super-absorbent polymer particles 56p is higher than that of the absorbent body 56 according to (I). As a result, the liquid retention ability of the absorbent body 56 is higher than that of the absorbent body 56 according to (I) and an effect of preventing reversion of liquid is also produced.

(III) In the absorbent body 56 constituted of softwood pulp fibers and hardwood pulp fibers, fibers with relatively long fiber length and large fiber width and fibers with relatively short fiber length and small fiber width as described above are to be combined and the resultant fiber density is about halfway between (I) and (II). Therefore, the absorption capacity by the fibers can be secured to a certain degree, the absorption speed of the absorbent body 56 can be retained, and the liquid retention ability of fibers due to capillary action can also be secured. In addition, the retainability of the super-absorbent polymer particles 56p between the pulp fibers 56f is also provided and the liquid retention ability by the super-absorbent polymer particles 56p can be further increased with respect to (I) and (II). As a result, liquid retention ability is also obtained while retaining the absorption capacity and the absorption speed of the absorbent body 56. In this manner, in the mode according to (III), since absorption capacity and absorption speed, and liquid retention ability, can both be secured, reversion prevention property can be improved.

Note that in (III) above, since a certain degree of bulkiness can be secured in the absorbent body 56 due to the absorbent body 56 containing softwood pulp fibers, a decline in texture of the obtained absorbent article can be suppressed. In addition, by containing softwood pulp fibers, operability when fabricating pulp sheets to fluff pulp (in other words, action to suppress scattering of powdery materials and adhesion of fibers to equipment) can also be secured. Favorable operability contributes to overall high productivity in manufacturing of absorbent articles.

In one preferable example of the hardwood pulp fiber-containing absorbent body 56, in all pulp fibers of the absorbent body 56, a percentage of pulp fibers with a fiber length of 0.5 mm or more and less than 1.1 mm is 40% or more by mass, and a percentage of pulp fibers with a fiber width of 10 µm or more and less than 35 µm is 90% or more by mass. In addition, preferably, the percentage of pulp fibers with a fiber length of 0.5 mm or more and less than 1.1 mm can be made 42.6% or more, more preferably 43% or more, and even more preferably 43.2% or more, 43.7% or more, and 45 or more. Furthermore, preferably, the percentage of pulp fibers with a fiber width of 10 µm or more and less than 35 µm can be made 90.6% or more, more preferably 91.0% or more, and even more preferably 91.2% or more and 91.4% or more. In this manner, due to the absorbent body 56 including specific pulp fiber lengths and specific pulp fiber widths in specific ranges, respectively, advantages (in particular, reversion prevention property) of the absorbent body 56 according to (III) described above can be further improved and made more reliable.

While an upper limit of the percentage of the pulp fibers with a fiber length of 0.5 mm or more and less than 1.1 mm among all pulp fibers 56f of the hardwood pulp fiber-containing absorbent body 56 is not particularly limited, due to a variability in fiber lengths attributable to pulp being a natural material, the percentage can be 80% by mass or less, 60% by mass or less, or 55% by mass or less. In addition, in a similar manner, while an upper limit of the percentage of the pulp fibers with a fiber width of 10 µm or more and less than 35 µm is also not particularly limited, the percentage can be 98% by mass or less, 95% by mass or less, or 94% by mass or less.

Note that the fiber length and the fiber width of pulp fibers can be measured using the measuring machine "VALMET FS5" according to JIS-P8226:2011 (ISO 16065-2:2007) "Pulps-Determination of fibre length by automated optical analysis".

Furthermore, in the hardwood pulp fiber-containing absorbent body 56, an average fiber length when pulp fibers with a fiber length of 0.5 mm or more and less than 1.1 mm are measured at 0.02 mm intervals (at a classification width of 0.02 mm) can be 0.5 to 0.8 mm and preferably 0.6 to 0.7 mm. Accordingly, in the absorbent body 56, a variability in a distribution of voids that may occur depending on the orientation direction of the pulp fibers can be suppressed and absorption speed can be secured.

In addition, in the hardwood pulp fiber-containing absorbent body 56, a standard deviation σ when pulp fibers with a fiber length of 0.5 mm or more and less than 1.1 mm are measured at 0.02 mm intervals (at a classification width of 0.02 mm) can be 0.58 mm or less, preferably 0.56 mm or less, more preferably 0.54 mm or less, and even more preferably 0.50 mm or less. Due to the standard deviation being within the range described above, the absorption capacity and the absorption speed of the absorbent body 56 can be improved and reversion prevention property of the absorbent body 56 can be improved.

Furthermore, the average fiber width when pulp fibers with a fiber width of 10 µm or more and less than 35 µm are measured at 1 µm intervals (at a classification width of 1 µm) can be 15 µm or more and 30 µm or less and preferably 18 µ or more and 27 µm or less. Setting the average fiber width within the range described above enables voids between fibers to be suitably retained and prevents a decline in absorption speed and, at the same time, avoids an excessive increase in a contact area between pulp fibers and prevents a binding force between fibers from excessively rising. Accordingly, defibrability during manufacturing can be set suitably. Note that when the defibrability of a pulp sheet is excessively low, there is a risk that excessive defibration may occur (defibration process may be excessively performed) during fabrication of fluff pulp and the absorption speed in the absorbent body produced by the creation of microfibers may decline.

The standard deviation σ when pulp fibers with a fiber width of 10 µm or more and less than 35 µm are measured at 1 µm intervals (at a classification width of 1 µm) can be 2.9 µm or less, preferably 2.8 µm or less, more preferably 2.75 µm or less, and even more preferably 2.6 µm or less and 2.59 µm or less. Setting the standard deviation within the range described above enables the absorption speed and the reversion prevention property to be improved.

As described above, the hardwood pulp fiber-containing absorbent body 56 in which variabilities of the distribution of fiber lengths in a specific range and the distribution of fiber widths in a specific range have been reduced has a favorable reversion prevention effect while securing absorption capacity and absorption speed despite containing hardwood pulp fibers.

While the raw material wood of the softwood pulp fibers included in the pulp fibers is not particularly limited, pines such as radiata pine and various types of cedar are suitably used.

In addition, while the raw material wood of the hardwood pulp fibers is also not particularly limited, acacia wood, eucalyptus wood, and the like are suitably used. Acacia wood is a material with characteristics of being strong and hard, with little shrinkage due to drying and high impact resistance, and pulp obtained from acacia wood also inherits its original properties and is a useful raw material pulp with high moisture absorption and drying properties. Eucalyptus wood belongs to the genus Eucalyptus and has long been widely used as a raw material pulp for paper production, especially Eucalyptus globulus, Eucalyptus grandis, Eucalyptus europhylla, Eucalyptus nitens, and Eucalyptus regnans, the pulp fiber obtained therefrom is rigid with a fiber lumen that is difficult to collapse, and the absorbent body can be made bulkier and lower in density by blending these pulp fibers. As hardwood pulp fibers, acacia wood is preferable because its fiber width is wider than that of eucalyptus wood, making acacia wood bulky, and there is less reduction in absorption volume and absorption speed when combined with softwood pulp fibers.

While a mass ratio of hardwood pulp fiber to other pulp fibers such as softwood pulp fiber in the hardwood pulp fiber-containing absorbent body 56 is not limited and can be, for example, 10/90 to 90/10, from the perspective of operability, the ratio is preferably 20/80 to 50/50, more preferably 25/75 to 38/62, even more preferably 25/75 or more and less than 38/62, even more preferably 26/74 to 36/64, and particularly preferably 28/72 to 35/65.

A content of acacia wood in the raw material wood of the hardwood pulp fiber or, in other words, the percentage of pulp fiber derived from acacia wood in the hardwood pulp fiber of the hardwood pulp fiber-containing absorbent body 56 is preferably 5 to 95% by mass, more preferably 5% by mass or more and less than 95% by mass, and even more preferably 20 to 93% by mass, 20 to 90% by mass, 25 to 85% by mass, and 50 to 80% by mass. Even with hardwood pulp fibers which have shorter fiber lengths than softwood pulp fibers and tend to be tightly packed together, and regardless of a direction in the fibers are oriented (for example, any of the length direction, the width direction, and/or the thickness direction of the obtained absorbent article), more uniform voids between the fibers can be secured.

In addition to the softwood bleached kraft pulp and the hardwood bleached kraft pulp described above, for example, soda pulp, sulfite pulp, chemi-thermo mechanical pulp, chemirefiner mechanical pulp, thermo-chemi mechanical pulp, and the like may be used as the pulp fibers that constitute the absorbent body 56. However, bleached pulp with sufficient lignin removed is preferred since the absorption amount and the absorption speed of the pulp fibers readily decrease if the lignin in the fibers is not sufficiently removed. When the other pulps described above are contained, the content of the other pulps is preferably 10% by mass or less with respect to all pulp raw materials.

In addition, the pulp fibers 56f included in the absorbent body 56 may include recycled pulp fibers, regardless of their type. For example, the pulp fibers 56f may contain recycled softwood pulp fibers or recycled hardwood pulp fibers. When using recycled pulp fibers, an amount of ash in the pulp fiber used in the absorbent body such as an amount of ash in fluff pulp is preferably as small as possible. In addition, if recycled pulp fibers are to be used, when pulp derived from the same raw material is compared before and after recycling, while whiteness of the recycled pulp may be lower or equal to that of virgin pulp (non-recycled pulp), equal whiteness is preferable.

When the absorbent body 56 is to be constituted of a plurality of layers, only a part of the layers may contain hardwood pulp fibers or all of the layers may contain hardwood pulp fibers. For example, when the absorbent body 56 is constituted of two layers, the upper layer 56a and the lower layer 56b, only the lower layer 56b may contain hardwood pulp fibers or only the upper layer 56a that can come into direct contact with skin may contain the hardwood pulp fibers.

In addition, although also depending on product usage, a basis weight of the pulp fibers 56f in the absorbent body 56 (when the absorbent body is constituted of a plurality of layers, basis weight of all of the plurality of layers) is 100 to 500 g/m², preferably 100 to 300 g/m², and more preferably 120 to 250 g/m². For example, in the case of a disposable diaper, the basis weight of the pulp fibers can be 100 to 300 g/m² and preferably 120 to 200 g/m². Furthermore, for example, in the case of a sanitary napkin, the basis weight of the pulp fibers in the absorbent body 56 (when the absorbent body is constituted of a plurality of layers, basis weight of all of the plurality of layers) is 150 to 500 g/m² and preferably 250 to 400 g/m². The basis weight of the pulp fibers may be uniformed or regions of which the basis weight is relatively high or relatively low may be formed.

### (Super-absorbent polymer particle)

The super-absorbent polymer particles 56p include "powders" in addition to "particles". As the super-absorbent polymer particles 56p, those used in disposable diapers of this type can be used as they are. While a pre-swelling particle diameter (particle diameter when liquid is not absorbed) of the super-absorbent polymer particles 56p is not particularly limited, the super-absorbent polymer particles 56p with a pre-swelling particle diameter of more than 150 µm and 850 µm or less are preferably 60% by mass or more of a total amount, more preferably 70% by mass or more of the total amount, and particularly preferably 80% by mass or more of the total amount. In addition, the super-absorbent polymer particles 56p with a pre-swelling particle diameter that is shorter than the average fiber length of the hardwood pulp fibers (for example, 600 µm or less and more preferably 500 µm or less) are preferably 60% by mass or more of a total amount, more preferably 70% by mass or more of the total amount, and particularly preferably 80% by mass or more of the total amount.

A particle diameter distribution of the super-absorbent polymer particles 56p can be determined by setting standard sieves (for example, standard sieves manufactured by Tokyo Screen Co., Ltd.) with apertures of 850 µm, 600 µm, 500 µm, 355 µm, 300 µm, 250 µm, and 150 µm as specified in JIS Z 8801 and a receiving tray, in this order from the top, on a sieve shaker (for example, Model AS 200 manufactured by RETSCH GmbH), feeding all of the super-absorbent polymer particles into the topmost sieve, and performing sieving. Shaking conditions are 50 Hz, amplitude: 0.5 mm, and shaking time: 10 minutes. In addition, sieving is performed at a temperature of 23 ± 2°C and a humidity of 50 ± 5%, and measurements are to be performed by storing samples in a same environment for at least 24 hours before sieving. Sieving is performed three times, and an average value of the three times is adopted as the mass on the sieve for each sieve. From the obtained mass of the super-absorbent polymer particles on each sieve and the total mass of the super-absorbent polymer particles (mass of all super-absorbent polymer particles), a content ratio (mass percentage) of a corresponding particle diameter range between each sieve (in other words, more than 850 µm, more than 600 µm and 850 µm or less, more than 500 µm and 600 µm or less, more than 355 µm and 500 µm or less, more than 300 µm and 355 µm or less, more than 250 µm and 300 µm or less, more than 150 µm and 250 µm or less, and 150 µm or less) can be determined. In addition, if a particle size accumulation curve is obtained based on the particle diameter distribution and the particle diameter corresponding to a median accumulated value (50%) of the accumulation curve is taken as the average particle diameter, the average particle diameter of the super-absorbent polymer particles is preferably within a range of 355 to 500 µm and more preferably within a range of 370 to 470 µm.

While materials for the super-absorbent polymer particles 56p can be used without any particular limitation, materials with a water absorption of 40 g/g or more are suitable. The super-absorbent polymer particles 56p can be starch-based, cellulose-based, or synthetic polymer-based (polyacrylate-based, polysulfonate-based, or maleic anhydridebased), and a starch-acrylic acid (salt) graft copolymer, a saponified product of a starch-acrylonitrile copolymer , a cross-linked sodium carboxymethyl cellulose, an acrylic acid (salt) polymer, and the like can be used. While a suitable shape for the super-absorbent polymer particles 56p is a powdery granular form that is normally used, other shapes can also be used.

As the super-absorbent polymer particles 56p, super-absorbent polymer particles with a water absorption speed of 70 seconds or less and, particularly, 40 seconds or less are suitably used. If the water absorption speed is too slow, reversion (liquid supplied into the absorbent body 56 returns to outside of the absorbent body 56) more readily occurs.

In addition, as the super-absorbent polymer particles 56p, super-absorbent polymer particles with a gel strength of 1000 Pa or more are suitably used. Accordingly, even when the absorbent body 56 is bulky, a sticky sensation after liquid absorption can be effectively suppressed.

A basis weight of the super-absorbent polymer particles 56p can be appropriately determined according to an absorption amount required by the usage of the absorbent body 56. Therefore, although it depends on the situation, the basis weight of the super-absorbent polymer particles 56p in the absorbent body 56 (when the absorbent body 56 is constituted of a plurality of layers, basis weight of all of the plurality of layers) is 50 to 350 g/m² and preferably 100 to 300 g/m². For example, in the case of a disposable diaper, the basis weight of the super-absorbent polymer particle 56p can be 50 to 300 g/m² and preferably 100 to 250 g/m². In addition, for example, in the case of a sanitary napkin, the basis weight of the super-absorbent polymer particles 56p in the absorbent body 56 (when the absorbent body 56 is constituted of a plurality of layers, basis weight of all of the plurality of layers) is 70 to 470 g/m² and preferably 140 to 240 g/m². When the absorbent body 56 is constituted of two layers, the basis weight of the super-absorbent polymer particles 56p in the lower layer 56b may differ from the basis weight of the super-absorbent polymer particles 56p in the upper layer 56a. In other words, the basis weight of the super-absorbent polymer particles 56p in the lower layer 56b may be more than the basis weight of the super-absorbent polymer particles 56p in the upper layer 56a or, conversely, the basis weight of the super-absorbent polymer particles 56p in the lower layer 56b may be less than the basis weight of the super-absorbent polymer particles in the upper layer 56a.

A ratio of the pulp fibers 56f and the super-absorbent polymer particles 56p in the absorbent body 56 is not particularly limited and, for example, pulp fibers 56f: super-absorbent polymer particles 56p can be 40:60 to 65:35 by weight.

A post-swelling average particle diameter of the super-absorbent polymer particles 56p is preferably 2,000 to 3,000 µm and more preferably 2,200 to 2,800 µm. The post-swelling average particle diameter of the super-absorbent polymer particles 56p means an average value of diameters of the super-absorbent polymer particles 56p in a swollen state after immersion in a saline solution for 60 minutes and draining for 15 minutes. The average value of diameters is obtained by observing a group of the super-absorbent polymer particles 56p in a swollen state using a microscope at a magnification of 50 times from one direction, measuring the maximum diameters of any 20 particles that appear entirely on a surface of the group of the super-absorbent polymer particles 56p, and calculating an arithmetic mean. In particular, if the post-swelling average particle diameter of the super-absorbent polymer particle 56p is larger than the average fiber length of the hardwood pulp fibers, the swelling of the super-absorbent polymer particles 56p is less likely to be inhibited (so-called gel blocking is less likely to occur) because the intertwining between the pulp fibers 56f is readily broken by the swelling of the super-absorbent polymer particles 56p and is, therefore, preferable.

### (Low-basis weight portion)

The absorbent body 56 may have an elongated low-basis weight portion 56L that extends in the front-back direction LD on both sides in the width direction WD in the crotch portion M or the like as in the example shown in Figs. 1 to 4 or, although not illustrated, need not have the low-basis weight portion 56L. The low-basis weight portion 56L means a portion of which the basis weight is low and does not include portions which are simply compressed in a thickness direction TD and of which the basis weight does not change such as a high-compression portion 51 to be described later. While the low-basis weight portion 56L can be a slit that penetrates in the thickness direction TD, the low-basis weight portion 56L is preferably a concave portion with a small accumulation of pulp fibers and super-absorbent polymer particles as in the illustrated example since an absorption amount can be readily secured. The concave portion may be formed on a front surface or a back surface of the absorbent body 56. Providing the low-basis weight portion 56L described above on the absorbent body 56 promotes bending of the absorbent body 56 along the low-basis weight portion 56L and improves fit of the absorbent element 50 in the crotch portion M. A total basis weight of the pulp fibers and the super-absorbent polymer particles in the low-basis weight portion 56L need only be smaller than a total basis weight of the pulp fibers and the super-absorbent polymer particles in portions other than the low-basis weight portion 56L and can be set to, for example, 0.1 to 0.5 times the total basis weight of the pulp fibers and the super-absorbent polymer particles in portions other than the low-basis weight portion 56L.

As long as the low-basis weight portion 56L has an elongated shape that extends in the front-back direction LD, the low-basis weight portion 56L may extend linearly in the front-back direction LD or may be curved so as to be positioned to the side the more toward both sides in the front-back direction LD as in the illustrated example. In addition, front and back ends of the low-basis weight portion 56L can have an appropriate shape and, for example, in addition to a linear shape as in the example shown in Fig. 12(a), a shape that bulges in a curved manner (semicircular shape or the like) as in the example shown in Fig. 8 may be adopted or, although not illustrated, corners of both end portions may be rounded and a middle portion may have a linear shape. A width m1 of the low-basis weight portion 56L can be determined as appropriate and can be, for example, 0.04 to 0.1 times a width n1 of a narrowest portion in the crotch portion M of the absorbent body 56 (in the case of a rectangle, this means the full width 56X). The width m1 of the low-basis weight portion 56L may be constant or may vary in a length direction thereof. A dimension and an arrangement of the low-basis weight portion 56L in the front-back direction LD may be determined as appropriate. For example, a dimension m2 of the low-basis weight portion 56L in the front-back direction LD can be 50 to 120% and more preferably 50 to 80% of the dimension of the crotch portion M in the front-back direction LD. In addition, the low-basis weight portion 56L may fit within the range of the crotch portion M or may extend out to a front side, a back side, or both front and back sides of the crotch portion M.

In addition to providing one each of the low-basis weight portion 56L on both sides in the width direction WD and providing one at center in the width direction WD in the crotch portion M as shown in Fig. 8, only one low-basis weight portion 56L can be provided at center as shown in Fig. 12(a) or only one each of the low-basis weight portion 56L can be provided on both sides in the width direction WD in the crotch portion M as shown in Fig. 12(b).

### (High-compression portion)

In the absorbent element 50, as shown in Figs. 3, 4, and 9 to 11, a plurality of high-compression portions 51 that are compressed in the thickness direction TD so as to be recessed into the absorbent body 56 from a surface of the absorbent element 50 are provided spaced apart. In addition, in an arrangement region of the plurality of high-compression portions 51 (a rectangular region which is constituted of sides respectively along the front-back direction LD and the width direction WD and which is circumscribed around all of the high-compression portions 51), portions other than the high-compression portions 51 are non-high-compression portions 52 which are thicker and have lower density than the high-compression portions 51. The high-compression portions 51 are high-density portions that have been compressed by pressing (direct pressure) to approximately equal thickness and are bottom portions of depressions. On the other hand, while the non-high-compression portions 52 are portions that are thicker and lower in density than the high-compression portions 51, even in the non-high-compression portions 52, density increases near a periphery of high-compression portions 51 the closer to the high-compression portions 51 due to the absorbent body 56 deforming as if pulled by a deformation of the high-compression portions 51. As long as the non-high-compression portions 52 are thicker and lower in density than the high-compression portions 51, a part of or all of the non-high-compression portions 52 may be compressed in the thickness direction TD at the same time as the formation of the high-compression portions 51 or before or after the formation of the high-compression portions 51. Preferably, the non-high-compression portions 52 do not have depressions on any one of or both of the front surface and the back surface of the absorbent element 50.

In addition to being provided across the entireties of the front-back direction and the width direction of the absorbent element 50 (in other words, an entire surface of the absorbent element 50 is an arrangement region of the high-compression portions 51), the high-compression portions 51 can also be provided in only one portion in the front-back direction or in only one portion in the width direction of the absorbent element 50. For example, although not illustrated, the high-compression portions 51 can be provided only in the intermediate region in the front-back direction LD including the crotch portion M or, conversely, instead of being provided in the intermediate region in the front-back direction LD including the crotch portion M, the high-compression portions 51 can be provided only in regions on both front and back sides of the intermediate region. In addition, while the high-compression portions 51 in the illustrated example are not depressed from a surface of a sheet positioned on a front side of the absorbent element (in the case of the illustrated example, the top sheet 30 and the intermediate sheet 40) to inside the absorbent body 56, the high-compression portions 51 may be depressed from the surface of the absorbent element 50 to inside the absorbent body 56 as a result of being pressed so as to become depressed into the absorbent body 56 from the sheet positioned on the front side of the absorbent element 50.

While a density of the absorbent body 56 in the high-compression portions 51 is not particularly limited, the density is preferably 60000 to 250000 g/m³ and more preferably 65000 to 200000 g/m³. On the other hand, while a density of the absorbent body 56 in the non-high-compression portion 52 is not particularly limited, the density is preferably 50000 to 200000 g/m³ and more preferably 55000 to 150000 g/m³. The density of the absorbent body 56 in the high-compression portions 51 and the non-high-compression portions 52 is measured by the following method.

### (Measurement method of density of absorbent body 56 in high-compression portions 51)

(1) Peel off at least one of the sheets on the front side and the back side of the absorbent element 50 so that the surface of the absorbent element 50 with the high-compression portions 51 is exposed, and measure an area of the high-compression portions 51 by the method described below.
(2) Measure the basis weight of the absorbent element 50.
(3) Cut a portion of the wrapping sheet 58 from the absorbent element 50 and measure the basis weight of the wrapping sheet 58.
(4) Determine the mass of the absorbent element 50 in the high-compression portions 51 from the measurement results of (1) and (2), determine the mass of the wrapping sheet 58 in the high-compression portions 51 from the measurement results of (1) and (3), and determine the mass of the absorbent body 56 in the high-compression portions 51 by subtracting the latter from the former.
(5) After freezing the absorbent element 50 with liquid nitrogen, measure the thickness of the absorbent body 56 by cutting the high-compression portions 51 across approximately the center in the thickness direction TD and observing the cut surface with a microscope.
(6) Assuming that the thickness of the absorbent body 56 in the high-compression portions 51 is constant, calculate a volume of the absorbent body 56 in the high-compression portions 51 by multiplying the area of the high-compression portions 51 in (1) above by the thickness of the absorbent body 56 in (5) above.
(7) Calculate the density of the absorbent body 56 in the high-compression portions 51 by dividing (4) above by (6) above.

### (Measurement method of density of absorbent body in non-high-compression portions)

(1) Peel off at least one of the sheets on the front side and the back side of the absorbent element 50 so that the surface of the absorbent element 50 with the non-high-compression portions 52 is exposed, and using a pen, mark five rectangular measurement object regions with predetermined dimensions in the non-high-compression portions 52. Measure the dimensions of the measurement object regions and calculate areas thereof.
(2) Measure the basis weight of the absorbent element 50.
(3) Cut a portion of the wrapping sheet 58 from the absorbent element 50 and measure the basis weight of the wrapping sheet 58.
(4) Determine the mass of the absorbent element 50 in the measurement object regions from the measurement results of (1) and (2), determine the mass of the wrapping sheet 58 in the measurement object regions from the measurement results of (1) and (3), and determine the mass of the absorbent body 56 in the measurement object regions by subtracting the latter from the former.
(5) After freezing the absorbent element 50 with liquid nitrogen, measure the thickness of the absorbent body 56 by cutting the measurement object regions across approximately the center in the thickness direction TD and observing the cut surface with a microscope.
(6) Assuming that the thickness of the absorbent body 56 in the measurement object regions is constant, calculate a volume of the absorbent body 56 in the measurement object regions by multiplying the area of the measurement object regions in (1) above by the thickness of the absorbent body 56 in (5) above.
(7) Calculate the density of the absorbent body 56 in the non-high-compression portions 52 by dividing (4) above by (6) above.

While an area of each high-compression portion 51 is not particularly limited, the area is preferably 2 to 25 mm² and more preferably 3 to 15 mm².

The high-compression portions 51 are high-density portions that have been compressed in the thickness direction TD by pressing (direct pressure) to approximately equal thickness and are bottom portions of depressions that are recessed from at least one of the front and back surfaces of the absorbent element 50. Such high-density portions have a property that makes it difficult for entanglement of the fiber to be broken even if the fiber expands due to liquid absorption. On the other hand, while the non-high-compression portions 52 are portions that are thicker and lower in density than the high-compression portions 51, even in the non-high-compression portions 52, density increases near a periphery of high-compression portions 51 the closer to the high-compression portions 51 due to the absorbent body 56 deforming as if pulled by a deformation of the high-compression portions 51. Consequently, capillary action is strongly expressed in the high-compression portions 51 and not only is liquid retention higher than the periphery but liquid is also attracted toward the high-compression portions 51. Therefore, if each of the high-compression portions 51 provided at intervals has a certain density and a certain area, in the absorbent body 56 containing hardwood pulp fibers, the strength of the absorbent body 56 is less likely to decrease after liquid absorption, and a suction force due to capillary action acts on a larger amount of excretory liquid, allowing the excretory liquid absorbed by the absorbent body 56 to spread over a wider area.

Although not illustrated, the absorbent element 50 can be compressed in the thickness direction TD so as to be depressed from both the front and back surfaces of the absorbent element 50 into the absorbent body 56, or the high-compression portions 51 can be compressed in the thickness direction TD so as to be depressed only from the back surface of the absorbent element 50 into the absorbent body 56, in which case a depression is to be formed on the back surface of the absorbent element 50. However, if the high-compression portions 51 are compressed in the thickness direction TD so as to be depressed from at least the front surface of the absorbent element 50 to within the absorbent body 56 as in the illustrated example, both the reversion prevention property and diffusivity are improved. In other words, capillary action is strongly expressed in the high-compression portions 51 and not only is liquid retention higher than the periphery but liquid is also attracted toward the high-compression portions 51 as described earlier, and excretory liquid collected by the high-compression portions 51 can be strongly retained in portions far from the skin. As a result, reversion is less likely to occur.

In addition, a shortest distance d4 to the closest other high-compression portion 51 can be 1 to 4 mm and more preferably around 1.5 to 3.5 mm. If the high-compression portions 51 are provided somewhat close together in this manner, the strength of the absorbent body 56 is less likely to decrease after liquid absorption and a suction force due to capillary action acts on a larger amount of excretory liquid, allowing the excretory liquid absorbed by the absorbent body 56 to spread over a wider area. In addition, when the high-compression portions 51 having a certain area are provided at such intervals, due to a reduction in the distance between pulp fibers 56f in the high-compression portions 51, entanglement of the pulp fibers 56f is strengthened, voids among the pulp fibers 56f that serve as migration paths of the super-absorbent polymer particles 56p decrease, contact portions of the pulp fibers 56f with respect to the super-absorbent polymer particles 56p increase, and the like, thereby inhibiting the migration of the super-absorbent polymer particles 56p within the absorbent body 56 and the migration of the super-absorbent polymer particles 56p to the outside of the absorbent body 56. In other words, retainability of the super-absorbent polymer particles 56p in the absorbent body 56 containing hardwood pulp fibers increases.

An area rate of the high-compression portions 51 in the arrangement region of the high-compression portions 51 (sum of areas of high-compression portions 51/ area of arrangement region of high-compression portions 51) is preferably 10 to 35% and more preferably 20 to 35%. In addition, a diameter of a largest inscribed circle 54 inscribed in the outline of the non-high-compression portions 52 is preferably 30 mm or less, more preferably 10 mm or less, even more preferably 6.5 mm or less, even more preferably 6 mm or less, and particularly preferably 5 mm or less. Furthermore, a lower limit of the diameter of the largest inscribed circle 54 inscribed in the outline of the non-high-compression portions 52 is preferably 2 mm, more preferably 3 mm, and particularly preferably 4 mm.

In this manner, arranging the high-compression portions 51 particularly densely produces a further improvement in liquid diffusivity described earlier. In addition, retainability of the super-absorbent polymer particles 56p also further improves. Furthermore, even in the non-high-compression portions 52, due to the absorbent body 56 deforming near a periphery of the high-compression portions 51 as if pulled by a deformation of the high-compression portions 51, an advantage in that the absorbent body 56 becomes thinner compared to when the high-compression portions 51 are absent is produced. From these perspectives, the area rate of the high-compression portions 51 is particularly preferably within the ranges described above. In addition, it is particularly preferable if the diameter of the largest inscribed circle 54 inscribed in the outline of the non-high-compression portions 52 is small so that the high-compression portions 51 are not sparsely arranged (so that the non-high-compression portions 52 do not continue long in all directions).

Dimensions and a shape of the high-compression portions 51 can be determined as appropriate. However, since the high-compression portions 51 are hard portions, the absorbent body 56 as a whole may lack flexibility or make a user feel as though a foreign body is mixed into the absorbent body 56 (foreign body sensation) if the dimensions of the high-compression portions 51 are excessively large, the shape of the high-compression portions 51 is excessively long in one direction, or the shape has an excessively intricate outline. In addition, when the dimensions of the high-compression portions 51 are excessively small, the shape of the high-compression portions 51 is excessively long in one direction, or the outline is excessively intricate, a problem in that the wrapping sheet 58 becomes susceptible to tearing arises when the wrapping sheet 58 is crepe paper. Therefore, a diameter of a largest inscribed circle 53 inscribed in the outline of the high-compression portions 51 is preferably 1 to 10 mm, more preferably 1 to 6 mm, even more preferably 2 to 5 mm, and particularly preferably 2.5 to 3.5 mm. In addition, a circumferential length of the outline of the high-compression portions 51 is preferably 1 to 15 times a circumferential length of the largest inscribed circle 53 inscribed in the outline of the high-compression portions 51, more preferably 1 to 5 times, even more preferably 1.0 to 2.5 times, even more preferably 1.0 to 2.0 times, and particularly preferably 1.0 to 1.6 times.

As an example, a major axis d1 (length of a long side of a smallest circumscribed rectangle) of each high-compression portion 51 can be about 4 to 9 mm, and a minor axis d2 (length of a short side of the smallest circumscribed rectangle) can be about 2 to 5 mm. When the lengths of four sides of the smallest circumscribed rectangle of each high-compression portion 51 are equal to each other, a length d3 of the sides can be about 2 to 5 mm.

While a shortest dimension of each high-compression portion 51 is not particularly limited, the shortest dimension is preferably longer than the average fiber length of the hardwood pulp fibers, more preferably 4 times the average fiber length or more, even more preferably 4 to 10 times the average fiber length, and particularly preferably 4 to 12 times the average fiber length. When the shortest dimension of each high-compression portion 51 is longer than the average fiber length of the hardwood pulp fibers, there are advantages in that the pulp will be less biased and shape retainability of the absorbent body is improved. In addition, the shortest dimension of each high-compression portion 51 is preferably longer than the post-swelling average particle diameter of the super-absorbent polymer particles 56p, more preferably 1.5 times the post-swelling average particle diameter or more, even more preferably 1.5 to 6 times the post-swelling average particle diameter, and particularly preferably 1.5 to 5 times the post-swelling average particle diameter. When the shortest dimension of each high-compression portion 51 is longer than the post-swelling average particle diameter of the super-absorbent polymer particles 56p, there are advantages in that retainability of the super-absorbent polymer particles can be increased and bias in absorption performance can be reduced.

An outline of the high-compression portions 51 is not particularly limited, and while a shape that has no inflection points and bending points such as a circle, an oval, or a rounded rectangle (including those in which either of the opposite sides are not straight lines but semicircles) is preferred, the outline may also be a triangle, a square, a cloud-shape, an X-shape, a V-shape, a U-shape, or the like.

In general, when improving a reversion prevention property, a mixing ratio of super-absorbent polymer particles with high liquid retention is often increased. In addition, in recent years, the mixing ratio of super-absorbent polymer particles is often increased for purposes such as making the absorbent body 56 thinner. However, increasing the mixing ratio of super-absorbent polymer particles may not only reduce the shape retainability of the high-compression portions 51 but may also make it difficult for the absorbent body 56 to deform near the periphery of the high-compression portions 51 in the non-high-compression portions 52 as if pulled by a deformation of the high-compression portions 51, and may make it difficult to improve a low reversion property and diffusivity. In addition, since a phenomenon (gel blocking) in which the super-absorbent polymer particles that have swelled after absorbing excretory liquid adhere to one another and inhibit the diffusion of the excretory liquid is more likely to occur, improving diffusivity may be difficult. Therefore, when the basis weight of the pulp fibers in the absorbent body 56 is 100 to 500 g/m², a ratio by weight of pulp fibers to super-absorbent polymer particles in the absorbent body 56 is preferably 45:55 to 65:35.

While a thickness 50t of the absorbent element 50 and a thickness 51t of the high-compression portions 51 can be determined as appropriate, insufficient compression may make it difficult to improve low reversion property and diffusivity. Therefore, when the range of basis weights of the pulp fibers and the range of ratios between the pulp fibers and the super-absorbent polymer particles described above are to be adopted, the thickness 50t of the absorbent element 50 (thickness of the non-high-compression portions 52) is preferably 3 to 13 mm. In this case, while the thickness 51t of the high-compression portions 51 (a minimum value when thickness varies) can be determined as appropriate, normally, the thickness 51t is preferably 60 to 90% of the thickness 50t of the absorbent element 50.

The high-compression portions 51 can be arranged in an appropriate regular or irregular pattern. For example, as the high-compression portions 51, a pattern in which the high-compression portions 51 provided in dotted line-shapes are arrayed in a grid as shown in Fig. 17, a pattern in which dotted high-compression portions 51 are arrayed in a staggered or matrix-like manner as shown in Figs. 18 and 19, and a pattern in which X-shaped high-compression portions 51 are arrayed in a matrix-like manner as shown in Fig. 20 can be exemplified. In addition, although not illustrated, a pattern in which linearly-contiguous high-compression portions are arrayed in a grid or a pattern in which high-compression portions with different sizes and shapes are arrayed spaced apart so as to represent a predetermined design may be adopted. In particular, when the high-compression portions 51 arrayed in dotted line-shapes or the linearly-contiguous high-compression portions 51 are arrayed in a grid, since movement of the super-absorbent polymer particles 56p of the non-high-compression portions 52 are kept within a region enclosed by the high-compression portions 51, an uneven distribution of the super-absorbent polymer particles 56p can be suppressed using fewer high-compression portions 51. For example, as in the example shown in Fig. 21, a pattern may be adopted in which a plurality of high-compression portions 51 with different areas are arrayed, with the area of the largest high-compression portion 51 being three times the area of the smallest high-compression portion 51 or more. The pattern shown in Fig. 21 includes a plurality of linear high-compression portions 51 with different lengths but the same width, in which the length of the longest high-compression portion 51 is three times the length of the shortest high-compression portion 51 or more. In addition, in the pattern of the example shown in Fig. 21, the high-compression portions 51 extending in the front-back direction LD, the high-compression portions 51 extending in an oblique direction, and the high-compression portions 51 having portions that extend in the front-back direction LD and portions extending obliquely from both front and back ends of the portions extending in the front-back direction LD are combined and arranged. Note that although dimensions of each portion are shown in Figs. 17 to 21, these dimensions are simply examples and it goes without saying that the dimensions can be individually modified as appropriate.

One preferable pattern of the high-compression portions 51 is the example shown in Figs. 11 and 16. In this example, when a virtual lattice formed by repetitively arraying first virtual straight lines 81 along a first direction at first intervals 81d in a second direction inclined at 80 to 90° clockwise in a plan view with respect to the first virtual straight lines 81 and repetitively arraying second virtual straight lines 82 along the second direction at second intervals 82d in the first direction is defined in the arrangement region of the high-compression portions 51 and a smallest virtual quadrangle 83 of which vertices are intersections of the first virtual straight lines 81 and the second virtual straight lines 82 is defined, the high-compression portions 51 are constituted of first high-compression portions 51a arranged at intersection positions of the first virtual straight lines 81 and the second virtual straight lines 82, second high-compression portions 51b respectively arranged between adjacent first high-compression portions 51a on the first virtual straight lines 81 and adjacent first high-compression portions 51a on the second virtual straight lines 82, and third high-compression portions 51c arranged at intersection positions of diagonals of the virtual quadrangles. In addition, the first high-compression portions 51a form circular shapes centered at intersections of the first virtual straight lines 81 and the second virtual straight lines 82, the second high-compression portions 51b form a rounded rectangle (or an oval) having a center of gravity at a midpoint of each side of the virtual quadrangle and having a major axis along each side, and the third high-compression portions 51c have a rounded rectangle (or an oval or a circular shape centered at the intersection of the diagonals of the virtual quadrangles) having a center of gravity at the intersection of the diagonals of the virtual quadrangle and having a major axis along the front-back direction LD. In this pattern, the diffusivity of the second high-compression portions 51b in a major axis direction (first direction and second direction) can be improved and since the second high-compression portions 51b are to have both a portion where the non-high-compression portions 52 are linearly continuous in the first direction and a portion where the non-high-compression portions 52 are linearly continuous in the second direction, the absorbent body 56 as a whole is readily deformed so as to conform to the body surface even if individual high-compression portions 51 harden.

When arraying the high-compression portions 51 along the virtual grid in the example shown in Figs. 11 and 16, while dimensions of each high-compression portion 51 can be determined as appropriate, configuring the first high-compression portions 51a so that diameters thereof are 1 to 4 mm, configuring the second high-compression portions 51b so that a length of the major axis is 3 to 6 mm and a length of the minor axis is equal to the diameter of the first high-compression portions 51a, configuring the third high-compression portions 51c so as to have the same dimensions and the same shape as the second high-compression portions 51b with the exception of an orientation of the major axis being along the front-back direction LD, and setting a shortest distance d4 between the first high-compression portions 51a and the second high-compression portions 51b that are adjacent to each other to 1 to 2 mm are preferable since not only reversion prevention property and liquid diffusivity but deformability of the absorbent body 56 is also improved. First interval 81d: second interval 82d is preferably within the range of 0.9:1.1 to 1.1:0.9 and, particularly, the first interval 81d and the second interval 82d are preferably equal to each other. The first interval 81d and the second interval 82d can be around 10 to 14 mm.

In addition, the virtual grid described above preferably has a diagonal grid shape of which the first virtual straight lines 81 is inclined at 40 to 50° clockwise with respect to the front-back direction LD in a plan view and the second virtual straight lines 82 is inclined at 40 to 50° counterclockwise with respect to the front-back direction LD in a plan view, thereby providing deformability of the absorbent body 56 when being worn and superior liquid diffusivity.

Specific examples of an array of the high-compression portions 51 are shown in Table 2.

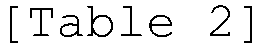

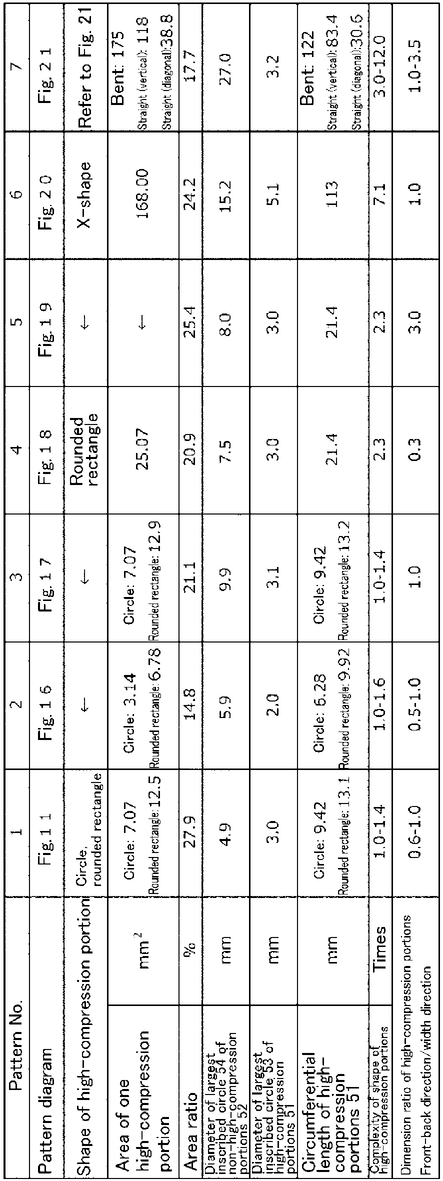

The absorbent element can be manufactured by known methods. For example, the absorbent element 50 having the high-compression portions 51 can be manufactured by performing: a first step of forming the absorbent body 56 by mixing and aggregating pulp fibers and super-absorbent polymer particles; a second step of forming a wrapped body 50P by wrapping an entirety of the absorbent body 56 with the wrapping sheet 58; and a third step of forming the high-compression portions 51 by passing, as shown in Fig. 22, the wrapped body 50P between an anvil roll 90 with a large number of protrusions 91 arranged at intervals in the same pattern as the high-compression portions 51 on an outer peripheral surface and a smooth roll 92 having a cylindrical surface (without the protrusions 91) opposing the anvil roll 90 and pressing portions of the wrapped body 50P that are sandwiched between the large number of protrusions 91 of the anvil roll 90 and the smooth roll 92. The high-compression portions 51 may be formed by heating one of or both of the anvil roll 90 and the smooth roll 92 or the high-compression portions 51 may be formed without any heating. In the third step, only the portions of the wrapped body 50P that are sandwiched between the large number of protrusions 91 of the anvil roll 90 and the smooth roll 92 may be pressed or the portions of the wrapped body 50P that are sandwiched between the large number of protrusions 91 of the anvil roll 90 and the smooth roll 92 may be pressed deepest while pressing the entirety of the wrapped body 50P.

Dimensions, a shape, and an arrangement of tip surfaces of the protrusions 91 can be similar to, for example, the dimensions, the shape, and the arrangement of the high-compression portions 51. A clearance (minimum distance between pressing positions) between the anvil roll 90 and the smooth roll 92 can be equal to or less than the thickness of the high-compression portions 51 and can be 0.5 to 1.5 mm as an example. While a pressing force when forming the high-compression portions 51 with the anvil roll 90 and the smooth roll 92 can be determined as appropriate, for example, the pressing force can be 0.2 to 0.4 MPa.

### (Wrapping sheet)

As the wrapping sheet 58, sheets with liquid permeability such as crepe paper, nonwovens, poly-laminated nonwovens, and sheets with small holes can be used. While the nonwovens used for the wrapping sheet 58 are not particularly limited, SMS nonwovens, SSMMS nonwovens, and the like in which at least one meltblown layer is sandwiched between a pair of front and back spunbonded layers can be suitably used. A material of the fibers is not particularly limited and, for example, polypropylene fiber, polyethylene/polypropylene bicomponent fiber, and the like can be used. While a basis weight of the wrapping sheet 58 can be determined as appropriate, the basis weight is desirably 5 to 40 g/m² and particularly desirably 10 to 30 g/m².

While the crepe paper used as the wrapping sheet is not particularly limited, the crepe paper desirably has a basis weight of 13 to 20 g/m² and particularly 14 to 18 g/m². In addition, an air permeance of the crepe paper measured in conformity with JIS P 8117: 2009 "Paper and board-Determination of air permeance and air resistance (medium range)- Gurley method" is desirably 1 to 15 seconds.

An average fiber length of the pulp constituting the crepe paper used for the wrapping sheet 58 may be less than twice the average fiber length of all pulp fibers constituting the absorbent body 56, but 1.5 to 3 times is preferable because of an advantage of increased material strength. The average fiber length of the pulp constituting the crepe paper used for the wrapping sheet 58 is more preferably 1.5 to 3 times the average fiber length of all pulp fibers constituting the absorbent body 56.

On the other hand, the crepe paper used for the wrapping sheet 58 generally has a stretch at break in the front-back direction LD of 20 to 35% and a stretch at break in the width direction WD of 4 to 8% (particularly, 5 to 7%) as specified in JIS P 8113: 2006. When the wrapping sheet 58 is such crepe paper, the crepe paper may tear when forming the high-compression portions 51 at edges of the high-compression portions 51 or between adjacent high-compression portions 51, but adopting a configuration in which the outline of the high-compression portions 51 has no inflection points or bending points, the diameter of the largest inscribed circle inscribed in the outline of the high-compression portions 51 is 2 to 5 mm, and a circumferential length of the outline of the high-compression portions 51 is 1 to 2.5 times the circumferential length of the largest inscribed circle inscribed in the outline of the high-compression portions 51 enables such tearing to be reduced.

An MD direction of the wrapping sheet 58 is preferably configured along the front-back direction LD due to ease of manufacture. In this case, since a fiber orientation of the wrapping sheet 58 is the front-back direction LD and a dry tensile strength in the width direction WD is significantly weaker than a dry tensile strength in the front-back direction LD, if the dimension of the high-compression portions 51 in the front-back direction LD is twice the dimension of the high-compression portions 51 in the width direction WD or more and particularly more than three times longer (in other words, the shape of the high-compression portions 51 is long in the front-back direction LD), the wrapping sheet 58 is likely to tear on both sides of the width direction WD. Therefore, the dimension of the high-compression portions 51 in the front-back direction LD is preferably less than twice and particularly 1.5 times the dimension of the high-compression portions 51 in the width direction WD or less since the wrapping sheet 58 is less likely to tear.

While a wrapping structure of the wrapping sheet 58 can be determined as appropriate, from the perspectives of ease of manufacture, prevention of leakage of super-absorbent polymer particles from edges of front and back ends, and the like, preferably, as in the illustrated example, the absorbent body 56 is wrapped in a cylindrical shape so as to surround the front and back surfaces and both side surfaces of the absorbent body 56, front and back edge portions are made to protrude from the front and back of the absorbent body 56, and overlapping portions of the wrapping and overlapping portions of the front and back protruding portions are joined by joining means such as a hot-melt adhesive, material welding, and the like.

In particular, as shown in Fig. 15, configuring the wrapping sheet 58 so as to have a front surface portion 58s positioned on a front side of the absorbent body 56, a first back-side portion 58a which is a portion continuing from the front surface portion 58s, extending around one side edge of the absorbent body 56, and reaching the back side of the absorbent body 56, and a second back-side portion 58b which is a portion continuing from the front surface portion 58s, extending around the other side edge of the absorbent body 56, and reaching the back side of the absorbent body 56, the first back-side portion 58a and the second back-side portion 58b having stacked portions 58W that overlap each other, and all the high-compression portions 51 being formed in the stacked portions 58W is preferable because the crepe paper on the formation surface of the high-compression portions 51 is doubled, becomes stronger, and is less likely to tear during the formation of the high-compression portions 51.

The shape retainability of the absorbent element 50 by the high-compression portions 51 is affected by the retainability of the high-compression portions 51 themselves, and the retainability of the high-compression portions 51 themselves is affected by the joining strength between the wrapping sheet 58 and the absorbent body 56 in the high-compression portions 51 and by the shape retainability of the absorbent body 56 itself. In addition, when the wrapping sheet 58 and the absorbent body 56 are bonded by hot-melt adhesives HM1 and HM2, more of the hot-melt adhesives HM1 and HM2 than usual are required to maintain sufficient joining strength between the wrapping sheet 58 and the absorbent body 56 and the shape retainability of the absorbent body 56 itself with an increase in surface area due to the formation of the high-compression portions 51. Therefore, the surface of the absorbent body 56 and the inner surface of the wrapping sheet 58 are preferably bonded via the hot-melt adhesives HM1 and HM2 of 5.0 to 20.0 g/m² and particularly 7.5 to 15.0 g/m² at least over an entire region having the high-compression portions.

For example, as shown in Fig. 9, when a depression due to the high-compression portions 51 is formed on the surface of the absorbent body 56, an amount of the hot-melt adhesive HM described above can be achieved by providing two layers of the hot-melt adhesives HM1 and HM2 on the back surface of the absorbent body 56 over the entire region having the high-compression portions 51. As shown in Fig. 13, such a structure can be manufactured by: after applying a first hot-melt adhesive HM1 over approximately the entire surface facing the absorbent body 56 on the inner surface of the wrapping sheet 58 in the expanded state, arranging the absorbent body 56 in an intermediate portion of the first hot-melt adhesive HM1 in the width direction WD in the wrapping sheet 58, and bonding the back surface of the absorbent body 56 and the wrapping sheet 58 via the first hot-melt adhesive HM1; next, after applying a second hot-melt adhesive HM2 to approximately the entire surface of the absorbent body 56, folding portions of the wrapping sheet 58 that protrude on both sides of the absorbent body 56 over the surface of the absorbent body 56 as shown in Fig. 14, and bonding the surface of the absorbent body 56 and the folded portion of the wrapping sheet 58 via the first hot-melt adhesive HM1 and the second hot-melt adhesive HM2; and bonding the overlapping portions of the wrapping sheet 58 via the first hot-melt adhesive HM1 and subsequently forming the grid-like high-compression portions 51 by embossing as shown in Fig. 9.

The absorbent element 50 may not have any other sheet layer such as paper or a nonwoven between the wrapping sheet 58 and the absorbent body 56 (naturally, the absorbent element 50 may have an adhesive layer) as shown in Fig. 9 or the like for the purpose of reducing cost, or the absorbent element 50 may have other sheet layers on at least one of the front side and the back side of the absorbent body 56.

### <Manufacturing method of absorbent body>

The absorbent body 56 can be manufactured by known methods. Fig. 25 schematically represents an example of an absorbent body manufacturing apparatus 100. In the absorbent body manufacturing apparatus, a pulp supplying unit 111 that supplies fiber materials including pulp is mainly provided upstream. The pulp supplying unit 111 may include a defibrator and a pulp sheet 110 is to be supplied to the defibrator.

When manufacturing the absorbent body 56 containing hardwood pulp fibers, the pulp sheet 110 including hardwood pulp fibers is supplied to the defibrator. In the defibrator, the pulp sheet 110 is defibrated into fibrous form by a mechanical process to obtain fluff pulp. Note that while the pulp sheet 110 may be in bale form or roll form, the roll form is preferable since productivity can be readily improved.

In addition, the apparatus used in the mechanical process for defibrating the pulp sheet 110 is not particularly limited. Known defibrators used when manufacturing absorbent articles such as disposable diapers can be used and defibrators that utilize frictional forces and shear forces as the mechanical process can be suitably used. Examples of defibrator systems include hammer-type defibrators, impacttype defibrators, roll-type defibrators, and jet stream-type defibrators.

The fluff pulp obtained by defibration is supplied to inside a duct 112. A polymer particle introducing unit 113 for supplying super-absorbent polymer particles may be provided midway along the duct 112. Therefore, the pulp fibers 56f (fluff pulp) and the super-absorbent polymer particles 56p are supplied to a fiber-stacking drum 115 after being mixed inside the duct 112. Absorbent body molds 115C are arranged in plurality spaced apart in a circumferential direction on an outer peripheral surface of the fiber-stacking drum 115. Due to rotation of the fiber-stacking drum 115, the absorbent body molds 115C are to sequentially receive the mixture of the pulp fibers 56f and the super-absorbent polymer particles 56p. Bottom surfaces of the absorbent body molds 115C are constituted of a perforated plate or a mesh plate which allows the mixture of the pulp fibers and the super-absorbent polymer particles to accumulate on the bottom surfaces of the absorbent body molds 115C by sucking gas through open holes. In addition, due to discharge of gas through the open holes, the absorbent body 56 can be supplied to conveying means 116 such as a conveyor belt. A negative pressure chamber and a positive pressure chamber are provided inside the fiber-stacking drum 115, and each chamber enables suction and discharge of gas, respectively.

A scuffing roll (or a brush) 114 may be provided at a downstream location in the duct 112. The scuffing roll 114 can scrape off excess absorbent body material that has been stacked and can make the surface of the absorbent body material accumulated on the absorbent body mold 115C uniform. In addition, the absorbent body 56 supplied to the conveying means 116 can be compressed in the thickness direction by compressing means 117. The compressing means 117 may be a pair of rolls as shown in Fig. 25 or a pair of pressing members that approach from both sides of the absorbent body 56. The compressing means 117 improves shape retainability of the absorbent body 56.

The absorbent body 56 is wrapped further downstream with the wrapping sheet 58 to become the absorbent element 50. A step of wrapping the absorbent body 56 with the wrapping sheet 58 can also be performed by known methods.

When stacking two layers of the absorbent body 56, as shown in Fig. 26, a manufacturing apparatus 120 including single-layer manufacturing units 101 and 102 for manufacturing respective layers included in the absorbent body 56 or, in the illustrated example, the upper layer 56a and the lower layer 56b can be used. Both single-layer manufacturing units 101 and 102 have a similar configuration to the manufacturing apparatus 100 (Fig. 25) for manufacturing single-layer absorbent bodies. The layers 56a and 56b of the absorbent body manufactured by the single-layer manufacturing units 101 and 102, respectively, are layered in the thickness direction and then further wrapped in their entireties with the wrapping sheet 58 to become the absorbent element.

### (Rising gather)

The rising gather 60 has a rising portion 68 that rises from a side portion of the interior body 200, and the rising portion 68 comes into contact with a range of the wearer from the gluteal portion, around the legs, to the buttock and prevents side leakage. The rising gather 60 can also be omitted as necessary. While the rising gather 60 in the illustrated example is configured such that a root-side portion 60B rises diagonally toward a central side in the width direction and a tip-side portion 60A relative to an intermediate portion rises diagonally toward outside in the width direction, the rising gather 60 is not limited thereto and modifications may be made as appropriate such as rising toward a central side in the width direction as a whole.

To describe this in greater detail, the rising gather 60 in the illustrated example is constructed by folding, a strip-shaped gather sheet 62 having a length equal to the length of the interior body 200 in the front-back direction, in the width direction WD at a tip portion so as to be folded in two and fixing a plurality of elongated gather elastic members 63 at intervals in the width direction WD in an elongated state along the longitudinal direction between the folded portion and nearby sheets. A base portion positioned on an opposite side to the tip portion (an end portion on an opposite side to the sheet folded portion in the width direction WD) of the rising gather 60 constitutes the root portion 65 fixed to the side portion of the interior body 200, and a portion other than the root portion 65 constitutes a body portion 66 (portion on the folded portion side) which extends out from the root portion 65. In addition, the body portion 66 has the root-side portion 60B that extends toward a central side in the width direction and the tip-side portion 60A that is folded back at a tip of the root-side portion 60B and that extends outward in the width direction. Furthermore, while both end portions in the front-back direction of the body portion 66 constitute fallen portions 67 that are fixed to surfaces of side portions of the top sheet 30 in a fallen state, an intermediate portion in the front-back direction positioned between the fallen portions 67 constitute the nonfixed rising portion 68 and the gather elastic member 63 along the front-back direction LD is fixed in an elongated state to at least a tip portion of the rising portion 68.

In the rising gather 60 configured as described above, the rising portion 68 rises so as to come into contact with the skin as indicated by arrows in Fig. 3 due to a contraction force of the gather elastic member 63. In particular, when the root portion 65 is positioned on a back side of the interior body 200, since the rising portion 68 rises so as to open outward in the width direction in the crotch portion and a vicinity thereof, the rising gather 60 comes into surface contact with the circumference of the legs and fit improves. The root portion 65 can also be fixed to a front side of the interior body 200 such as surfaces of both side portions of the top sheet 30.

In a bent structure in which the body portion 66 is constituted of the root-side portion 60B that extends toward the central side in the width direction and the tip-side portion 60A that is folded back at the tip of the root-side portion 60B and that extends outward in the width direction as in the rising gather 60 according to the illustrated example, the tip-side portion 60A and the root-side portion 60B are joined in a fallen state at the fallen portion 67 and, at the same time, the root-side portion 60B is joined to the top sheet 30 in a fallen state. For the joining of opposing surfaces in the fallen portion 67, at least one of means that involve a hot-melt adhesive by various application methods and means that involve material welding such as heat sealing or ultrasonic sealing can be used. In this case, the joining of the root-side portion 60B and the top sheet 30 and the joining of the tip-side portion 60A and the root-side portion 60B may be performed by the same means or performed by different means. For example, preferably, the joining of the root-side portion 60B and the top sheet 30 is performed using a hot-melt adhesive and the joining of the tip-side portion 60A and the root-side portion 60B is performed by material welding.

As the gather sheet 62, flexible nonwovens with excellent uniformity and hiding properties such as spunbonded nonwovens (SS, SSS, and the like), SMS nonwovens (SMS, SSMMS, and the like), and meltblown nonwovens subjected to water repellent treatment using silicone or the like as necessary can be suitably used. A fiber basis weight of the nonwovens in this case is preferably around 10 to 30 g/m². In addition, although not illustrated, a waterproof film can also be interposed between parts of the gather sheet 62 folded in two.

Thread rubber or the like can be used as the gather elastic member 63. When using spandex thread rubber, a thickness thereof is preferably 470 to 1240 dtex and more preferably 620 to 940 dtex. A stretch rate in an attached state of the gather elastic member 63 is preferably 150 to 350% and more preferably 200 to 300%. The number of the gather elastic members 63 is preferably 2 to 6 and more preferably 3 to 5. A suitable arrangement interval of the gather elastic members 63 is 3 to 10 mm. Adopting such a configuration makes it easier for the gather elastic members 63 to come into surface contact with skin in the range where the gather elastic members 63 are arranged. The gather elastic members 63 may be arranged not only on the tip side but also on the root side.

In the rising portion 68 of the rising gather 60, for bonding of the inside layer and the outside layer of the gather sheet 62 and fixing of the gather elastic members 63 sandwiched between the inside layer and the outside layer, at least one of fixing means that involve a hot-melt adhesive by various application methods and fixing means that involve material welding such as heat sealing or ultrasonic sealing can be used. Since bonding entire surfaces of the inside layer and the outside layer of the gather sheet 62 results in loss of flexibility, portions other than the adhesive portion of the gather elastic member 63 are preferably left unbonded or weakly bonded. In the illustrated example, a configuration is adopted in which by applying a hot-melt adhesive only to outer peripheral surfaces of the gather elastic members 63 using application means such as a comb gun or a SureWrap nozzle and sandwiching the gather elastic members 63 between the inside layer and the outside layer of the gather sheet 62, the gather elastic members 63 can be fixed to the inside layer and the outside layer of the gather sheet 62 and the inside layer and the outside layer of the gather sheet 62 can be fixed to each other using only the hot-melt adhesive applied to the outer peripheral surfaces of the gather elastic members 63.

In a similar manner, for the fixing of the fallen portion 67, at least one of means that involve a hot-melt adhesive by various application methods and means that involve material welding such as heat sealing or ultrasonic sealing can be used.

### (Side flap)

As shown in Figs. 1 to 4 and the like, a side flap 70 that extends to the side of the absorbent body 56 is provided in both side portions of the interior body 200, and a side stretchable region SG that stretches in the front-back direction is preferably formed in the side flap 70. The side flap 70 according to the illustrated example has one or a plurality of elongated side elastic members 73 provided spaced apart from each other along the front-back direction LD, a first sheet layer 71 facing an outside of the side elastic members 73, and a second sheet layer 72 facing an inside of the side elastic members 73.

A sheet material that constitutes the first sheet layer 71 and the second sheet layer 72 is not particularly limited and appropriate nonwovens such as nonwovens that can be used in the rising gather 60 described above or the exterior bodies 12F and 12B described above can be selected. In the illustrated example, as will be described later, the first sheet layer 71 and the second sheet layer 72 are formed by extending the gather sheet 62 of the rising gather 60. In this case, front and back ends of the side flap 70 match front and back ends of the rising gather 60 (in other words, in this case, front and back ends of the interior body 200).

The side elastic member 73 is also not particularly limited and elongated elastic members similar to the gather elastic member 63 described earlier can be used. A stretch rate in an attached state of the side elastic member 73 is preferably 150 to 350% and more preferably 200 to 270%. The number of the side elastic members 73 is preferably 2 to 16 and more preferably 6 to 10. A suitable arrangement interval of the side elastic members 73 is 5 to 10 mm.

The side elastic member 73 is fixed to the first sheet layer 71 and the second sheet layer 72. For bonding of the first sheet layer 71 and the second sheet layer 72 and fixing of the side elastic member 73 sandwiched between the first sheet layer 71 and the second sheet layer 72, fixing means that involve the hot-melt adhesive HM by various application methods and fixing means that involve material welding such as heat sealing or ultrasonic sealing can be used. Since a large joining area of the first sheet layer 71 and the second sheet layer 72 results in loss of flexibility, portions other than the adhesive portion of the side elastic member 73 are preferably left unbonded or weakly bonded. In the illustrated example, a configuration is adopted in which by applying a hot-melt adhesive HM only to the outer peripheral surfaces of the side elastic members 73 using application means such as a comb gun or a SureWrap nozzle and sandwiching the side elastic members 73 between the first sheet layer 71 and the second sheet layer 72, fixing of the side elastic members 73 to the first sheet layer 71 and the second sheet layer 72 and fixing of the first sheet layer 71 and the second sheet layer 72 to each other are performed using only the hot-melt adhesive HM applied to the outer peripheral surfaces of the side elastic members 73.

In addition, in the illustrated example, the sheet material that constitutes the first sheet layer 71 and the sheet material that constitutes the second sheet layer 72 are folded back at a side edge of the side flap 70 and, at the same time, the folded-back portions are fixed to the back surface of the liquid-impermeable sheet 11 (sealed). In addition to using the hot-melt adhesive HM as in the illustrated example, the fixing can be performed by material welding.

The side flap 70 can also be omitted.

### <Example of preparing pulp sheet for manufacturing absorbent body>

Using the materials listed in Table 1, pulp sheets No. 1 to 20 containing hardwood pulp fibers were prepared according to the following procedure.

### (Digestion step)

### (1) Hardwood

Acacia and eucalyptus were mixed to the mass ratios listed in Table 1, white liquor with a predetermined sulfidity was added, and the mixture was digested in a rotary autoclave at a liquor to wood ratio of 6.0 (L/kg) and a maximum temperature of 170°C to obtain hardwood unbleached kraft pulp with a Kappa number of approximately 16.

### (2) Softwood

White liquor with a predetermined sulfidity was added to Japanese cedar, and the mixture was cooked in a rotary autoclave at a liquor to wood ratio of 6.0 (L/kg) and a maximum temperature of 170°C to obtain softwood unbleached kraft pulp with a Kappa number of approximately 30.

### (Washing step)

After the unbleached pulp was diluted to a pulp concentration of approximately 2%, No. 2 filter paper (manufactured by ADVANTEC) was laid on a Buchner funnel to dehydrate the pulp concentration to approximately 12%. Subsequently, the unbleached pulp was sandwiched between square filter papers (manufactured by ADVANTEC) and pressurized in a press to dehydrate the unbleached pulp to a pulp concentration of 33%. This washing step was performed twice.

### (Bleaching step)

### (1) Hardwood

The unbleached kraft pulp was subjected to ozone bleaching at 45°C with an ozone gas at 8.1% concentration for approximately 2 minutes. For the ozone bleaching, ozone gas generated by a laboratory ozone generator PSA Ozonizer SGA-01A-PSA4, manufactured by SUMITOMO PRECISION PRODUCTS Co., Ltd., was used. Furthermore, the pulp after ozone bleaching was bleached with chlorine dioxide at a chlorine dioxide additive rate of 1.5%, a pulp concentration of 10% by mass, and at 70°C to obtain hardwood bleached kraft pulp. Note that between the respective bleaching steps, a dilution/washing step in which after diluting to a pulp concentration of approximately 2%, No. 2 filter paper (manufactured by ADVANTEC) was laid on a Buchner funnel to dehydrate the pulp concentration to approximately 12% was performed twice.

### (2) Softwood

The unbleached kraft pulp was bleached with chlorine dioxide at a chlorine dioxide additive rate of 1.5%, a pulp concentration of 10% by mass, and at 70°C for 30 minutes. Subsequently, alkaline hydrogen peroxide bleaching was performed at an alkali additive rate of 1%, a hydrogen peroxide additive rate of 0.3%, a pulp concentration of 10% by mass, and at 70°C for 120 minutes. Subsequently, an alkali treatment was performed at a sodium hydroxide additive rate of 0.2%, a pulp concentration of 10% by mass, and at 70°C for 120 minutes. Subsequently, chlorine dioxide bleaching was performed at a chlorine dioxide additive rate of 0.3%, a pulp concentration of 10% by mass, and at 70°C for 150 minutes to obtain softwood bleached kraft pulp. Note that between the respective bleaching steps, a dilution/washing step in which after diluting to a pulp concentration of approximately 2%, No. 2 filter paper (manufactured by ADVANTEC) was laid on a Buchner funnel to dehydrate the pulp concentration to approximately 12% was performed twice.

### (Papermaking step)

The obtained hardwood bleached kraft pulp and softwood bleached kraft pulp were not beaten but diluted to concentrations of approximately 2% and adjusted to the combination ratios listed in Table 2. Then, the adjusted pulp slurries were used to make hand-rolled sheets (pulp sheets for fluff pulp) with a basis weight of 800 g/m² using a semiautomatic sheet machine (manufactured by KUMAGAI RIKI KOGYO Co., Ltd.).

### <Fabrication of absorbent article>

Using each pulp sheet, an absorbent body was fabricated using an apparatus such as that shown in Fig. 25, respectively. An amount of super-absorbent polymer added to the flaff pulp was 9.3 g for 7.7 g of fluff pulp. Using the obtained absorbent bodies, disposable diapers (taped diapers, size L) were fabricated without providing the high-compression portions 51 described earlier.

### <Measurement of fiber length and fiber width of pulp fibers>

A fiber length and a fiber width of the hardwood unbleached kraft pulp according to each example after the cooking step and the washing step were measured. The fiber length and the fiber width were measured using the fiber length measuring machine "VALMET FS5," manufactured by Valmet, according to JIS-P8226:2011 (ISO 16065-2:2007) "Pulps-Determination of fibre length by automated optical analysis". The fiber analyzer "VALMET FS5" is an apparatus capable of measuring the length and width of pulp fibers by image analysis of diluted pulp fibers as they pass through a measurement cell inside the fiber analyzer. From the obtained measurements, a percentage by mass of pulp fibers with a fiber length of 0.5 mm or more and less than 1.1 mm and a percentage by mass of pulp fibers with a fiber width of 10 µm or more and less than 35 µm among all pulp fibers were calculated. An average fiber length and a standard deviation σ when pulp fibers with the fiber length of 0.5 mm or more and less than 1.1 mm were measured at 0.02 mm intervals (at a classification width of 0.02 mm) and an average fiber length and a standard deviation σ when pulp fibers with the fiber width of 10 µm or more and less than 35 µm were measured at 1 µm intervals (at a classification width of 1 µm) were calculated. Note that the average values described above (values of average fiber lengths and average fiber widths) are arithmetic means. In addition, the standard deviation described above is calculated from n = 10000 or more measured based on the fiber analyzer. Calculation results are shown in Table 3.

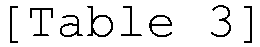

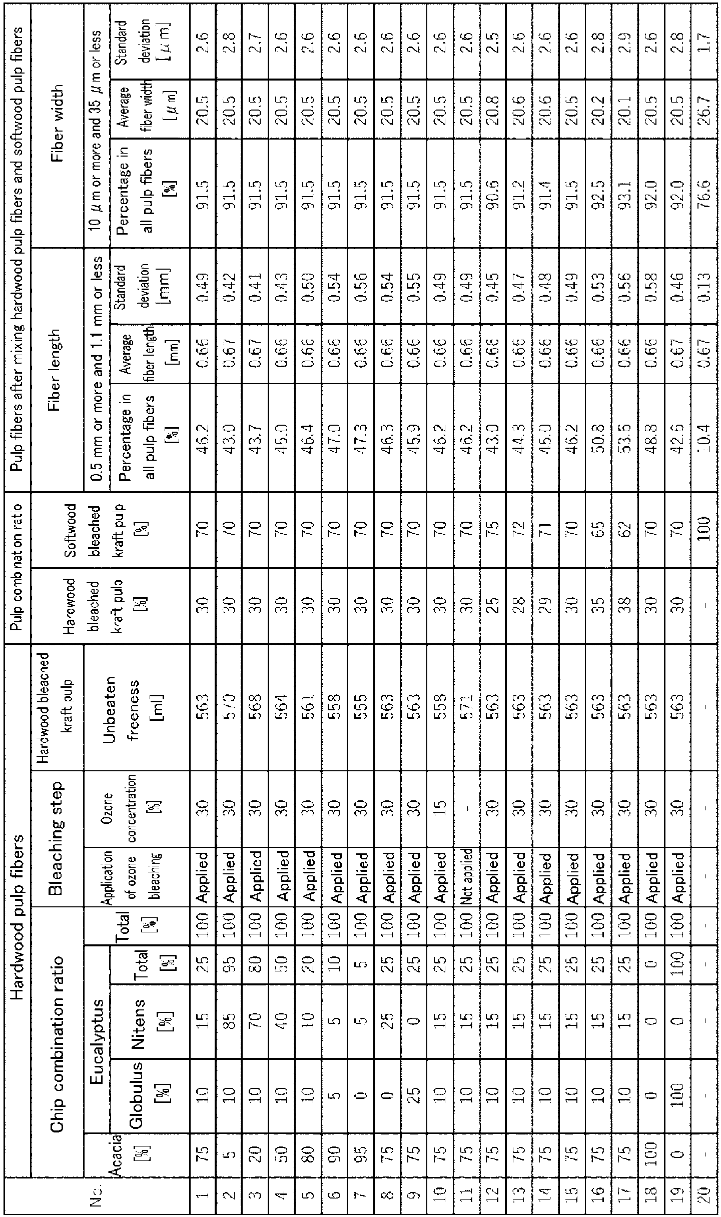

### <Evaluations>

Evaluations were performed as follows. Evaluation results are shown in Table 4.

### (Density)

The density of the pulp sheet for fluff pulp according to each example was measured according to "Paper and board-Determination of thickness, density and specific volume" described in JIS-P8118 (2014).

### (Calculation of burst index)

The burst index of the pulp sheet for fluff pulp according to each example was calculated as a value [kPa·m²/g] obtained by dividing a bursting strength [kPa] measured according to JIS-P8131 (2009) by the basis weight.

### (Evaluation of defibrability)

The defibrability of the pulp sheet for fluff pulp according to each example was evaluated based on the burst index described above. The evaluation criteria were the following five levels. When the evaluation is A, B, C, and D, the defibrability in the pulp sheet for fluff pulp is good, and among these levels, the defibrability in the case of A is excellent. In addition, while the level D is slightly inferior, the level does not pose any problems in terms of practical use.
A: Burst index is 1.2 kPa·m²/g or more and 1.5 kPa·m²/g or less.
B: Burst index is 1.6 kPa·m²/g or 1.1 kPa·m²/g.
C: Burst index is 1.7 kPa·m²/g or 1.0 kPa·m²/g.
D: Burst index is 1.8 kPa·m²/g or 0.9 kPa·m²/g.
E: Burst index is 1.9 kPa·m²/g or more or 0.8 kPa·m²/g or less.

### (Measurement of Klemm water absorptiveness)

The Klemm water absorptiveness of the pulp sheet for fluff pulp according to each example was measured according to "Paper and board-Determination of water absorptiveness(capillary rise)-Klemm method" described in JIS-P8141 (2004). More specifically, with respect to each pulp sheet for fluff pulp with a basis weight of 200 g/m², a height [mm] was measured 1 minute after the start of measurement, and a water absorption speed [mm/min] between 1 and 2 minutes after the start of measurement was calculated based on the heights [mm] at 1 minute and 2 minutes after the start of measurement.

### (Evaluation of water absorptiveness)

Based on the value of the height 1 minute after the start of measurement described above, the water absorptiveness (absorption performance) of the sheet for flap pulp was evaluated by the following criteria.
A: Height 1 minute after start of measurement according to Klemm water absorptiveness is 29 mm or more and 40 mm or less.
B: Height 1 minute after start of measurement according to Klemm water absorptiveness is 27 mm or more and less than 29 mm.
C: Height 1 minute after start of measurement according to Klemm water absorptiveness is 25 mm or more and less than 27 mm.
D: Height 1 minute after start of measurement according to Klemm water absorptiveness is less than 25 mm.

### (Evaluation of water absorptionspeed)

Based on the value of the water absorption speed between 1 minute and 2 minutes after the start of measurement described above, the water absorption speed of the sheet for flap pulp was evaluated by the following criteria.
A: Water absorption speed between 1 minute and 2 minutes after start of measurement according to Klemm water absorptiveness is 9 mm/minute or more.
B: Water absorption speed between 1 minute and 2 minutes after start of measurement according to Klemm water absorptiveness is 7 mm/minute or more and less than 9 m/minute.
C: Water absorption speed between 1 minute and 2 minutes after start of measurement according to Klemm water absorptiveness is 5 mm/minute or more and less than 7 mm/minute.
D: Water absorption speed between 1 minute and 2 minutes after start of measurement according to Klemm water absorptiveness is more than 3 mm/minute and less than 5 mm/minute.
E: Water absorption speed between 1 minute and 2 minutes after start of measurement according to Klemm water absorptiveness is 3 mm/minute or less.

### (Evaluation of workability when manufacturing fluff pulp)

The workability when manufacturing fluff pulp was evaluated by cutting the pulp sheet for fluff pulp according to each example with a basis weight of 200 g/m² into 2 cm × 2 cm, crushing the cut pulp sheet with a commercial mixer (Mixer TM856 manufactured by TESCOM CO.,LTD.), and examining a degree of paper dust generated during crushing. Note that the amount of paper dust deposited during crushing increases from E toward A. The evaluation was made according to the following five levels. Evaluations of levels A, B, C, and D mean workability is good.
A: Paper dust scatters when crushing the pulp sheet and pulp barely accumulates on the mixer wall.
B: Paper dust scatters when crushing the pulp sheet and pulp slightly accumulates on the mixer wall.
C: Paper dust scatters when crushing the pulp sheet and a small amount of pulp accumulates on the mixer wall.
D: Paper dust scatters when crushing the pulp sheet and, while pulp accumulates on the mixer wall to a certain extent, the accumulation is not problematic.
E: Paper dust scatters when crushing the pulp sheet, a lot of pulp accumulates on the mixer wall, and the level is unacceptable for actual use.

### (Evaluation of inhibition of skin rash by absorbent article)

A total of 220 testers were asked to use 10 of any of No. 1 to 22 of the disposable diapers fabricated as described above in each example. In other words, with respect to the disposable diaper according to one example, evaluations were made by 10 testers. In addition, the number of diapers in which rash occurred was evaluated as a percentage. The evaluation was made according to the following five levels. Evaluations of A, B, C and D mean that the inhibitory effect on skin rash is better than No. 20, which has a same configuration as an existing product.
A: Rash occurrence is much better than No. 20.
B: Rash occurrence is better than No. 20.
C: Rash occurrence is slightly better than No. 20.
D: Rash occurrence is barely better than No. 20 and practically usable.
E: Rash occurrence is equivalent to No. 20 and level is the same as existing product.

Note that an effect of inhibiting reversion of the absorbent body and an absorption capacity of the absorbent body are related to the rash inhibition effect.

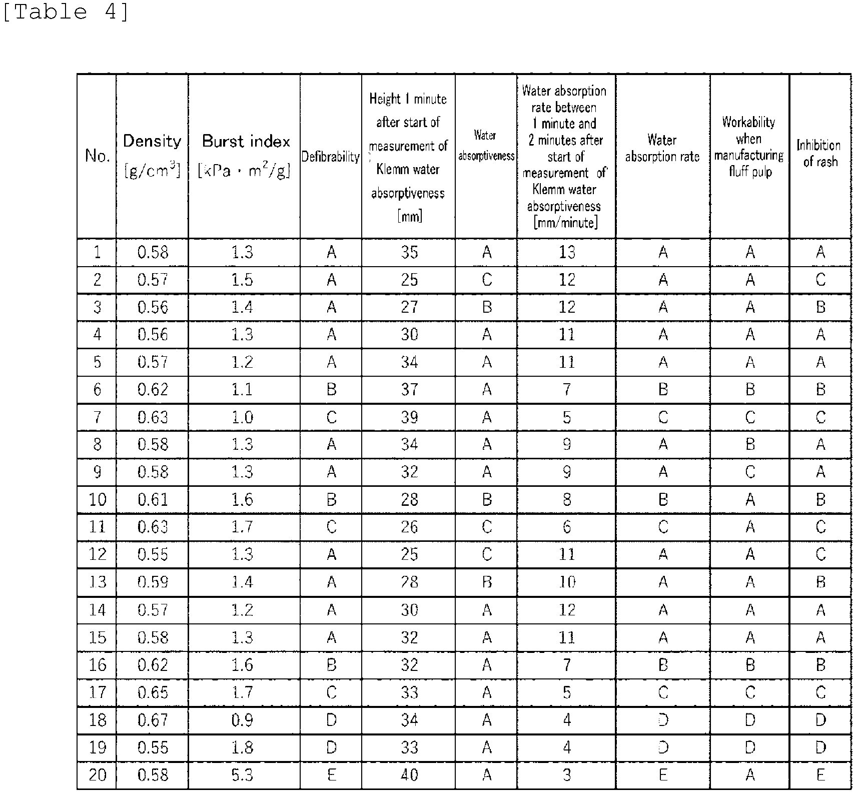

Tables 3 and 4 show that No. 1 to 19, which contain hardwood pulp fibers and softwood pulp fibers, and in which the percentage of pulp fibers with a fiber length of 0.5 mm or more and less than 1.1 mm in all pulp fibers is 42.6% or more, and the percentage of pulp fibers with a fiber width of 10 µm or more and less than 35 µm is 90.6% or more, produced favorable results particularly with respect to defibrability, workability when manufacturing fluff pulp, and a rash inhibition effect in absorbent articles.

### <Effect confirmation test 1>

Samples of pant-type disposable diapers (with similar structures to Figs. 1 to 6) including absorbent elements under various conditions shown in Table 5 were fabricated to perform: a measurement of thickness of the absorbent element, a test of reversion under pressure, a measurement of diffusion area, an absorbent body strength test, and a polymer fallout test. Note that the basis weight of the wrapping sheets, the dry tensile strength of the wrapping sheets, and the stretch at break of the wrapping sheets in Table 5 are measurements of materials before forming the high-compression portions.

Dimensions of each portion of samples 1 and 3 were as follows.
Length d3 of first high-compression portion 51a: 3 mm
Major axis d1 of second high-compression portion and third high-compression portion: 4.82 mm
Minor axis d2 of second high-compression portion and third high-compression portion: 3 mm
First interval 81d: 12.63 mm
Second interval 82d: 12.63 mm
Intersection angle between first virtual straight line 81 and second virtual straight line 82: 90 degrees

In samples 1 and 3, the high-compression portions were formed across the entire surface of the absorbent element under pressure of 0.4 MPa. In addition, the samples 2 and 4 constituted comparative examples without high-compression portions. Furthermore, conditions other than the specifications shown in Table 5 were commonly adopted by all samples.

### (Test of reversion under pressure)

The present test was conducted using a blue test solution prepared by dissolving approximately 5 g of Food Blue No. 1 (Brilliant Blue FCF) (packing: powder) manufactured by Kiriya Chemical in approximately 300 ml of artificial urine, according to the following procedure.
(1) After cutting the side seal 12A to open the front body F and the back body B of each sample, the sample was placed on a horizontally-installed flat plate in an orientation in which the front surface (surface having depressions of the high-compression portions) faced upward, and four corners of the sample were fixed to the flat plate with adhesive tape so that the sample was fixed in a state in which the interior body 200 was deployed flat.
(2) An injection cylinder (constituted of an acrylic cylinder portion with an inner diameter of 23 mm, an outline of 25 mm, and a height of 100 mm, and a square flange portion 100 mm long × 100 mm wide × 2 mm thick extending around the cylinder portion) was placed over a center position (center in the width direction and center in the front-back direction) of the absorbent body on the sample surface and held vertically.
(3) A graduated cylinder was used to inject 50 cm³ of the blue test solution described above into a top opening of the injection cylinder at a rate of 7 cm³/second, and waited from the start of injection until the artificial urine was no longer present on the sample surface (completely absorbed), at which point the injection cylinder was removed.
(4) The injection tube was removed and allowed to stand for 30 minutes.
(5) Following (4) above, steps (2) through (4) above were performed once again.
(6) Following (5) above, steps (2) and (3) above were performed once again.
(7) 20 minutes after the artificial urine from the third injection in (6) above was completely absorbed, a 5 kg weight was placed on the injection site of the artificial urine and left there for 10 minutes. The weight had a 100 mm long x 100 mm wide square flat base and was placed on the sample surface so that a longitudinal direction and a transverse direction of the weight were aligned with the front-back direction and the width direction of the sample and, at the same time, the center of the weight was positioned over the center of the absorbent body.
(8) A weight (g) of 30 sheets of Qualitative Filter Paper No. 2 (100 mm long × 100 mm wide square) manufactured by ADVANTEC was measured to the third decimal place on an electronic balance.
(9) Following (7) above, 30 sheets of the filter paper described above were stacked and sandwiched between the weight and the sample surface with centers and orientations aligned, and after 20 seconds, all of the sheets of filter paper were removed and its weight (g) was measured to the second decimal place, and the measured weight before absorption in (5) above was subtracted from the measured weight after absorption to determine the reversion amount.

### (Measurement of diffusion area)

Following the test of reversion under pressure described above, while keeping the sample fixed on the horizontally-installed flat plate, a ruler was arranged alongside the sample and a still image of the sample and the ruler was photographed from directly above it, the photographed image data was loaded to image analysis software "Fiji" (Fiji Is Just ImageJ), and an area of a blue portion on the sample surface was measured by the following procedure.
(1) Fiji was started up and an image to be measured was loaded by selecting "File" and then "Open".
(2) The "Straight" icon was selected and aligned with two points on the ruler's scale in the image.
(3) Analyze" and "Set Scale" were selected in that order, and actual dimensions between the two points (read from the ruler's scale) aligned in (2) above were entered in "Known distance" and the unit was entered in "Unit of length".
(4) "Image," "Type," and "8-bit" were selected in that order to convert the image into 8-bit.
(5) "Image," "Adjust," and "Threshold" were selected in that order to display the Threshold selection screen, the Threshold was set to 150 to 210 (Default Red), and "Dark background" and Don't reset range were checked.
(6) "Analyze" and "Set Measurements" were selected in that order, "Area," "Mean gray value," "Min & Max gray value," and "Limit to threshold" were checked, and OK was selected.
(7) "Polygon selections" was selected and a liquid diffusion area within the ranged indicated in red in (5) above was enclosed.
(8) "Analyze" and "Measure" were selected in that order to display "Results," and the 'Area' item was adopted as the diffusion area ("Unit of length" was set to cm, and the unit of area was set to cm²).

### (Absorbent body strength test)

(1) After cutting the side seal 12A to open the front body F and the back body B of each sample, the sample was placed on a horizontally-installed flat plate in an orientation in which the front surface (surface having depressions of the high-compression portions) faced upward, and four corners of the sample were fixed to the flat plate with adhesive tape so that the sample was fixed in a state in which the interior body 200 was deployed flat.
(2) Injection cylinders (each constituted of an acrylic cylinder portion with an inner diameter of 23 mm, an outline of 25 mm, and a height of 100 mm, and a square flange portion 100 mm long × 100 mm wide × 2 mm thick extending around the cylinder portion) were placed at a center position (center in the width direction and center in the front-back direction) of the absorbent body and a position 100 mm rearward from the center position, respectively, and held vertically.
(3) A graduated cylinder was used to inject 90 cm³ of the blue test solution described above into the top opening of the injection cylinder at a rate of 7 cm³/second, and waited from the start of injection until the artificial urine was no longer present on the sample surface (completely absorbed), at which point a 300 g weight was placed on the flange portion of each injection cylinder to uniformly apply a load to the flange portion.
(4) After the weight of (3) above was placed and left there for 5 minutes, the two weights and the rearward injection cylinder were removed, a graduated cylinder was used to inject 90 cm³ of the blue test solution described above into the top opening of the central injection cylinder at a rate of 7 cm³/second, and waited from the start of injection until the artificial urine was no longer present on the sample surface (completely absorbed), at which point a 300 g weight was placed on the flange portion of the central injection cylinder to uniformly apply a load to the flange portion.
(5) After the weight of (4) above was placed and left there for 5 minutes, the sample was removed from the flat plate, mounted on a pivot plate 303 of an impact tester 300 to be described later, and repeatedly impacted at 5-second intervals. Note that an outer surface of the front exterior body 12F of a sample was attached to a hook material 303F of the pivot plate 303 so that the front-back direction of the sample 320 is along an up-down direction in an initial state with the pivot plate 303 facing vertically downward.
(6) During impacting of (5) above, a tester observed the shape of the absorbent body of the sample 320 from the front with respect to the impact surface 301S, and after the tester recognized that the absorbent body had a tear, the tester interrupted impacting after each impact to measure the width of the tear with a ruler, and recorded the number of impacts until the width of the tear reached 3 cm as the absorbent body strength.

### (Impact tester)

The impact tester 300 is shown in Figs. 27 and 28. The impact tester 300 has: a support 310 constituted of a rectangular parallelepiped-shaped frame installed on a floor surface; a light box 301 attached to the middle of a back surface portion 311 of the support 310 in the up-down direction; a first pivot shaft 302 provided at a distance above the light box 301 in the back surface portion 311 and extending in an approximately horizontal and transverse direction; the pivot plate 303 with a flat plate shape which is supported so as to be forwardly and reversely rotatable between a state of facing vertically downward with one end portion fixed to the first pivot shaft 302 and a state rotated toward a front side from the state of facing vertically downward to face a horizontal direction; a second pivot shaft 304 concentrically provided with a center line that is an extension in a lateral direction of the center line of rotation of the first pivot shaft 302; a sample gripping arm 305 which extends in a radial direction from the second pivot shaft 304 and is supported by the second pivot shaft 304 so as to be forwardly and reversely rotatable; and a drive source (servo motor) 306 for rotating the sample gripping arm 305 forward and in reverse between a state of facing vertically downward and a state rotated toward a front side from the state of facing vertically downward to face an approximately horizontal direction. The light box 301 has a transparent impact surface 301S on a front surface and light from an internal illumination (white LED) 301L is transmitted through the impact surface 301S and emitted in a front direction. In addition, the pivot plate 303 has the hook material 303F of a mechanical fastener attached to approximately an entire surface to be a front side in an initial state of facing vertically downward, and the outer surface of the sample 320 can be attached to the hook material 303F. A tip portion of the sample gripping arm 305 has a clamping portion 307 for gripping a lower end portion of the sample 320 hanging from the pivot plate 303 facing vertically downward and, while maintaining this state, releasing the grip after the lower end portion of the sample 320 rotates to the front side to an approximately horizontal direction, and a drive source (not illustrated) for driving the clamping portion 307. Reference sign 307C denotes a pair of sandwiching portions for sandwiching the sample 320 from both front and back sides of the lower end portion of the sample 320.

In the present impact tester 300, after attaching the sample 320 to the pivot plate, in an initial state in which no external force is applied, the sample 320 and the pivot plate 303 face approximately vertically downward and the outer surface of the sample 320 comes into contact with the impact surface 301S of the light box 301 as shown by a solid line in Fig. 27. In addition, the sample gripping arm 305 faces approximately vertically downward and the clamping portion 307 in the tip portion opens. Here, when a test operation is started, the clamping portion 307 automatically closes and grips the lower end portion of the sample 320 as indicated by an arrow and a double-dotted line in the enlarged portion in Fig. 27, and due to the sample gripping arm 305 rotating until it becomes approximately horizontal to the front side as shown by the arrow and the double-dotted line in Fig. 27 while maintaining this state, the lower end portion of the sample 320 also faces approximately the same direction. Next, as indicated by the arrow and the double-dotted line in the enlarged portion in Fig. 27, due to the clamping portion 307 automatically opening, the sample 320 and the pivot plate 303 lose a restraining external force and rotate freely around the first pivot shaft 302 by their own weight, and a back surface of the sample 320 impacts against the impact surface 301S of the light box 301. Subsequently, the sample gripping arm 305 returns to its initial state. During the test operation, the impact operation (grip, rotate, open, and impact) of the sample 320 is repeated at set time intervals. By stopping the test operation, the sample 320 and the pivot plate 303 face approximately vertically downward and the sample 320 stops in a posture along the impact surface 301S of the light box 301. At this point, the tester facing the front surface of the sample 320 is able to visually recognize a shape of the absorbent body due to illumination light (difference in amount of transmission) from the light box 301 that passes through the sample 320. In particular, the outline of the absorbent body having absorbed the blue test solution described earlier can be readily visually recognized.

### (Polymer fallout test)

(1) After cutting the side seal 12A to open the front body F and the back body B of each sample, the back end portion of the interior body was cut along the back end of the absorbent body to expose the back end of the absorbent body. Note that since the back exterior body 12B of the present sample has the folded-back portion 12r that covers up to the end portion of the interior body 200 on the side of the waist opening WO, the folded-back portion 12r was detached using a cold spray and cut off.
(2) The outer surface of the front exterior body 12F of the sample was attached to the hook material 303F of the pivot plate 303 so that the front-back direction of the sample 320 is along an up-down direction in the initial state described earlier with the pivot plate 303 of the impact tester 300 facing vertically downward. In addition, a collection tray (not illustrated) was placed at a position 10 cm below the impact surface 301S to catch the super-absorbent polymer particles. Subsequently, impacting was repeated 30 times at 5-second intervals.
(3) After the impacting of (2) above, a weight of the fallen material in the collection tray was measured and recorded as an amount of polymer fallout.

Test results are shown in Table 5.

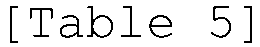

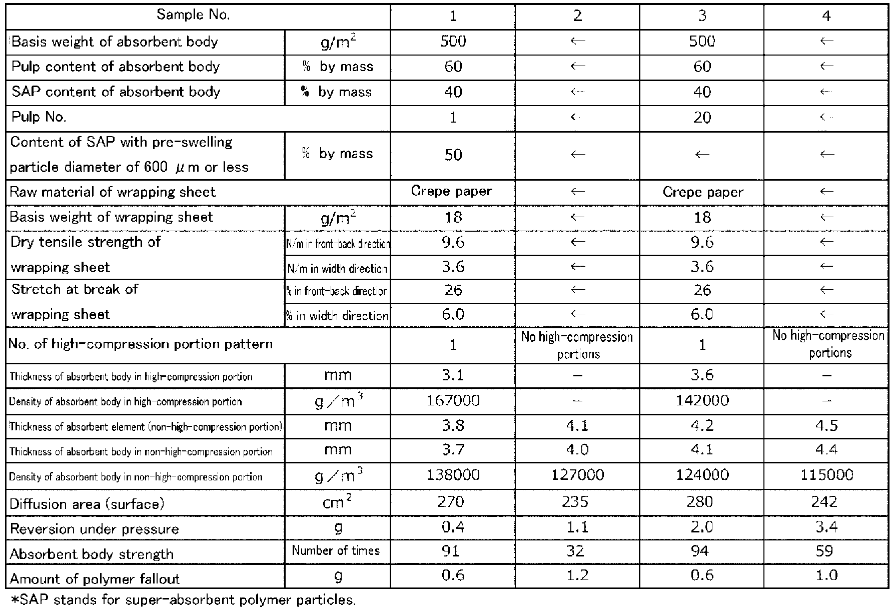

Sample 1, which is equipped with an absorbent body containing hardwood pulp fibers and in which high-compression portions are formed in the absorbent element, had a higher absorbent body strength than Sample 2, which is the same as Sample 1 with the exception of the absence of the high-compression portions, and had the same level of absorbent body strength as Sample 3 which is equipped with an absorbent body constituted solely of softwood pulp fibers. In addition, the diffusion area of Sample 1 was significantly larger than that of Sample 2 and was the same level as Sample 3 which is equipped with an absorbent body constituted solely of softwood pulp fibers. Furthermore, Sample 1 produced a notable result in that not only was the amount of reversion smaller than that of Sample 2, the amount of reversion was significantly small among all samples. In addition, the amount of polymer fallout of Sample 1 was smaller than that of Sample 2 and was the same level as Sample 3 which is equipped with an absorbent body constituted solely of softwood pulp fibers. Furthermore, as is apparent from a comparison between Samples 1 and 2 and a comparison between Samples 3 and 4, the thickness of the absorbent element was reduced due to the formation of the high-compression portions, and as is apparent from a comparison between Samples 1 and 3, the thickness of the absorbent element was further reduced due to the inclusion of hardwood pulp fibers.

### <Explanation of terms in specification>

The following terms in the specification have the following meanings unless otherwise described in the specification.

The "front-back direction" means the direction denoted by reference sign LD in the drawings (vertical direction), the "width direction" means the direction denoted by WD in the drawings (left-right direction), and the front-back direction and the width direction are orthogonal to each other.

The "front side" means a side closer to the skin of the wearer when being worn, and the "back side" means a side farther from the skin of the wearer when being worn.

The "front surface" means a surface closer to the skin of the wearer when being worn, and the "back surface" means a surface farther from the skin of the wearer when being worn.

The "stretch rate" means a value when a natural length is considered 100%. For example, a stretch rate of 200% is synonymous with an elongation factor of 2.

"Artificial urine" is a mixture of urea: 2 wt%, sodium chloride: 0.8 wt%, calcium chloride dihydrate: 0.03 wt%, magnesium sulfate heptahydrate: 0.08 wt%, and ion exchanged water: 97.09 wt%.

The "gel strength" is measured as follows. 1.0 g of super-absorbent polymer is added to 49.0 g of artificial urine and stirred with a stirrer. The generated gel is left in a constant temperature and humidity chamber at 40°C × 60% RH for 3 hours, then brought back to room temperature and the gel strength is measured with a curdmeter (Curdmeter-MAX ME-500, manufactured by I.techno Engineering).

The "basis weight" is measured as follows. After pre-drying the specimen or the test piece, the specimen or the test piece is left in the test room or apparatus under standard conditions (test site is at temperature 23 ± 1°C, relative humidity 50 ± 2%) to reach a constant volume. Pre-drying refers to bringing the specimen or the test piece to a constant volume at a temperature of 100°C. Note that fibers with an official regain of 0.0% need not be pre-dried. From the test piece having reached a constant volume, a specimen with dimensions of 100 mm × 100 mm is cut out using a sampling mold plate (100 mm × 100 mm). The weight of the specimen is measured and multiplied by 100 to calculate the weight per square meter, which is adopted as the basis weight.

The "thickness" of thick members such as the absorbent body 56 and the absorbent element 50 is measured using a thickness measuring instrument (Peacock, Dial Thickness Gauge, Model H (measuring range: 0 to 10 mm, measuring element: 10 mm-diameter circular pressure plane, measuring force: approximately 1.7 N, pressure: approximately 21.7 KPa)) manufactured by OZAKI MFG. CO., Ltd. by horizontally placing the test member and the thickness measuring instrument. The thickness of the absorbent element 50 (non-high-compression portions 52) is determined as follows. Among the non-high-compression portions 52 of the absorbent element 50, 10 regions where an inscribed circle inscribed in the outline of the non-high-compression portions 52 is the largest are selected, and the thickness measured by aligning the center of the measuring element of the thickness measuring instrument described above with the center of the inscribed circle in the regions is adopted as the thickness of the absorbent element 50 (non-high-compression portions 52).

The "thickness of the high-compression portions 51 is determined by subtracting a depth of the high-compression portions 51 from the thickness of the absorbent element 50 (non-high-compression portions 52).

The "depth" of the high-compression portions 51 is measured using the one-shot 3D measuring macroscope VR-3200 or its equivalent, observation application "VR-H2V," and analysis application "VR-H2A," all manufactured by KEYENCE CORPORATION, or their equivalent software. As a general rule, a magnification and a viewing area for measurement are set to 12 times and 24 mm × 18 mm, respectively, but the magnification and the viewing area can be changed depending on the size of the high-compression portions 51. Specifically, when measuring depth, the observation application described above is started up, a sample is placed on the microscope stage so that the high-compression portions 51 to be measured are positioned in a field of view, and the "Measure" button is pressed. Next, the analysis application described above is run and the measured 3D information is analyzed. An analysis procedure is described below with reference to Fig. 23. Using the analysis application described above, a depth profile (measured primary profile) at a line segment Q1 passing through the center (or center of gravity if non-circular) of one high-compression portion 51 in an image portion (XY portion in the drawing) shown from a planar viewpoint is obtained. Positions where the curve becomes almost flat or convex upward with the strongest curve in a "contour curve Q2" of the image portion (XZ portion in the drawing) shown in a cross-sectional viewpoint obtained by removing a surface roughness component with a wavelength shorter than Ac: 800 µm (where λc is the "filter defining a boundary between a roughness component and a waviness component" as described in JIS-B0601 "3.1.1.2") from the primary profile of the depth profile using a low-pass filter are adopted as the two boundary points P1 and P2, and a minimum value of heights in a range sandwiched by the boundary points P1 and P2 is determined. Furthermore, an average of the values of height of the boundary points P1 and P2 is adopted as a maximum value of the heights. The depth of the high-compression portion 51 can be determined by subtracting the minimum value of the heights from the maximum value of the heights. In addition, the two boundary points P1 and P2 are selected visually. In making the selection, the image in the planar viewpoint of the high-compression portion 51 being measured may be used as a reference. The measurement described above is performed with respect to any 10 high-compression portions 51 designed to have the same dimensions and shape, and results thereof are averaged.

Dimensions such as the diameter and intervals of the high-compression portions 51 are measured using the one-shot 3D measuring macroscope VR-3200 or its equivalent, observation application "VR-H2V," and analysis application "VR-H2A," all manufactured by KEYENCE CORPORATION, or their equivalent software. As a general rule, a magnification and a viewing area for measurement are set to 12 times and 24 mm × 18 mm, respectively, but the magnification and the viewing area can be changed depending on the size of the high-compression portions 51. Specifically, when measuring the dimensions, the observation application described above is started up, a sample is placed on the microscope stage so that the high-compression portions 51 to be measured are positioned in a field of view, and the "Measure" button is pressed. Next, the analysis application described above is run and the measured 3D information is analyzed.

An analysis procedure of the diameter of the high-compression portion 51 is described below with reference to Fig. 23. Using the analysis application described above, a depth profile (measured primary profile) at a line segment Q1 passing through the object high-compression portion 51 in any diameter direction in an image portion (XY portion in the drawing) shown from a planar viewpoint is obtained. Intersections with a straight line Q3 parallel to the X axis positioned at +0.25 mm from a lowest point 51L in a "contour curve Q2" of the image portion (XZ portion in the drawing) shown in a cross-sectional viewpoint obtained by removing a surface roughness component with a wavelength shorter than Ac: 800 µm (where λc is the "filter defining a boundary between a roughness component and a waviness component" as described in JIS-B0601 "3.1.1.2") from the primary profile of the depth profile using a low-pass filter are taken as P3 and P4, and a distance X1 between the intersections P3 and P4 in the X axis direction is adopted as the diameter of the high-compression portion 51. The measurement described above is performed with respect to any 10 high-compression portions 51 designed to have the same dimensions and shape, and results thereof are averaged.

In addition, an analysis procedure of the interval between adjacent high-compression portions 51 is described below with reference to Fig. 23. Using the analysis application described above, a depth profile (measured primary profile) at a line segment Q1 passing through two high-compression portions 51 adjacent in a direction of the object interval in an image portion shown from a planar viewpoint is obtained. Intersections with the straight line Q3 parallel to the X axis positioned at +0.25 mm from the lowest point 51L in a "contour curve Q2" of the image portion (XZ portion in the drawing) shown in a cross-sectional viewpoint obtained by removing a surface roughness component with a wavelength shorter than Ac: 800 µm (where λc is the "filter defining a boundary between a roughness component and a waviness component" as described in JIS-B0601 "3.1.1.2") from the primary profile of the depth profile using a low-pass filter are taken as P3, P4, P5, and P6, and a distance X2 in the X axis direction between the two adjacent intersections P4 and P5 in the middle is adopted as the interval of the high-compression portions 51. The measurement described above is performed with respect to any 5 pairs of adjacent high-compression portions 51 designed to have the same dimensions and shape, and results thereof are averaged.

The "thickness" of thin sheets such as nonwovens is automatically measured using an automatic thickness measuring instrument (Handy Compression Measurement Program KES-G5) under the conditions of load: 0.098 N/cm² and pressurized area: 2 cm².

The "area" of the high-compression portions 51 is measured using the one-shot 3D measuring macroscope VR-3200 or its equivalent, observation application "VR-H2V," and analysis application "VR-H2A," all manufactured by KEYENCE CORPORATION, or their equivalent software. As a general rule, a magnification and a viewing area for measurement are set to 12 times and 24 mm × 18 mm, respectively, but the magnification and the viewing area can be changed depending on the size of the high-compression portions 51. Specifically, when measuring the area, the observation application described above is started up, a sample is placed on the microscope stage so that the high-compression portions 51 to be measured are positioned in a field of view, and the "Measure" button is pressed. Next, the analysis application described above is run and the "Volume/Area" button is pressed to transition to the volume/area measurement screen in order to analyze the measured 3D information. On the volume/area measurement screen, after pressing the "Concave portion" button and then the "Set measurement region" button, the "Minimum Height" button in "Auxiliary Tool 2" is pressed to designate a region so as to include the one object high-compression portion 51, and a lowest point in the displayed region is selected. Next, after selecting Automatic Extraction (Level)" in "Element Tools," the lowest point is designated as a reference point, a height of the intersection setting is set to +0.25 mm, and the "OK" button is pressed.

Returning to the volume/area measurement screen, a value displayed as an area of a portion from the designated lowest point in the region to the height of 0.25 mm is adopted as the area of the high-compression portion 51. The measurement described above is performed with respect to any 5 high-compression portions 51 designed to have the same dimensions and shape, and results thereof are averaged.

The "area rate" means a ratio of an area of an object portion per unit area, expressed as a percentage of a total area of the object portion (for example, the high-compression portion 51) in the entire object region (for example, the back surface of the absorbent element 50) divided by the area of the object region. When determining the area rate of the high-compression portions 51, a rectangular region circumscribed around all of the high-compression portions 51 is considered the object region, and a total area of the high-compression portions 51 is to be determined from the areas of the high-compression portions 51 described below. When five or more of the high-compression portions 51 designed to have the same dimensions and shape are provided, all areas of the high-compression portions 51 of the design are to be an average value measured by the area measurement method of the high-compression portions 51 described above. On the other hand, when the dimensions and shapes of the high-compression portions 51 are irregular or the number of high-compression portions 51 designed to have the same dimensions and shape is less than five, the area of each of the high-compression portions 51 is to be measured by the area measurement method of the high-compression portions 51 described above (without determining an average value).

"Water absorptiveness" is measured according to JIS K7223-1996 "Testing method for water absorption capacity of super absorbent polymers".

"Water absorption speed" is defined as a "time to endpoint" when JIS K7224-1996 "Testing method for water absorption speed of super absorbent polymers" is performed using 2 g of a super-absorbent polymer and 50 g of a normal saline solution.

The "expanded state" means a state of flattened expansion without contraction (including any contraction such as contraction by elastic members) or sagging.

The dimensions of each portion mean the dimensions in the expanded state and not in a natural length state, unless otherwise stated.

If there is no description of environmental conditions with respect to a test or a measurement, the test or the measurement is to be performed in a laboratory or an apparatus under standard conditions (the test site shall be at a temperature of 23 ± 1°C and a relative humidity of 50 ± 2%).

### Industrial Applicability

The present invention can be used for absorbent articles having absorbent bodies in general including disposable diapers such as pant-type disposable diapers, tape-type disposable diapers, and pad-type disposable diapers, as well as sanitary napkins and pet diapers.

### Reference Signs List

- 11: Liquid-impermeable sheet
- 12A: Side seal
- 12B: Back exterior body
- 12E: Waist extension portion
- 12F, 12B: Exterior body
- 12F: Front exterior body
- 12S, 12H: Sheet material
- 13: Cover nonwoven
- 16, 19: Under-waist elastic member
- 17: Waist elastic member
- 18: Unnecessary elastic member
- 20: Interior body joint
- 200: Interior body
- 30: Top sheet
- 40: Intermediate sheet
- 50: Absorbent element
- 50P: Wrapped body
- 51: High-compression portion
- 51a: First high-compression portion
- 51b: Second high-compression portion
- 51c: Third high-compression portion
- 52: Non-high-compression portion
- 56: Absorbent body
- 56f: Pulp fiber
- 56p: Super-absorbent polymer
- 56L: Low-basis weight portion
- 58: Wrapping sheet
- 60: Rising gather
- 60A: Tip-side portion
- 60B: Root-side portion
- 62: Gather sheet
- 63: Gather elastic member
- 67: Fallen portion
- 68: Rising portion
- 70: Side flap
- 71: First sheet layer
- 72: Second sheet layer
- 73: Side elastic member
- 81: First virtual straight line
- 82: Second virtual straight line
- 90: Anvil roll
- 91: Protrusion
- 92: Smooth roll
- A1: Non-stretchable region
- A2: Stretchable region
- B: Back body
- C: Gluteal cover portion
- F: Front body
- HM: Hot-melt adhesive
- HM1: First hot-melt adhesive
- HM2: Second hot-melt adhesive
- L: Intermediate region
- LD: Front-back direction
- LO: Leg opening
- M: Crotch portion
- SG: Side stretchable region
- T: Lower torso region
- TD: Thickness direction
- U: Under-waist portion
- W: Waist portion
- WD: Width direction
- WO: Waist opening

## Claims

1. An absorbent article having a crotch portion, comprising:
an absorbent element comprising an absorbent body provided in a range in a front-back direction including the crotch portion, and a wrapping sheet that wraps the absorbent body, wherein
the absorbent body is made of mixing and aggregating pulp fibers including hardwood pulp fibers and super-absorbent polymer particles,
a percentage of particles of which a pre-swelling particle diameter is smaller than an average fiber length of the hardwood pulp fibers in the super-absorbent polymer particles is 60% by mass or more,
the absorbent element is provided with a plurality of spaced high-compression portions that are compressed in a thickness direction so as to be recessed into the absorbent body from at least one of front and back surfaces of the absorbent element,
portions other than the high-compression portions in an arrangement region of the plurality of high-compression portions are non-high-compression portions that are thicker and lower in density than the high-compression portions,
a shortest distance from each of the plurality of high-compression portions to the closest other high-compression portion is 1 to 4 mm, and
an area of each of the high-compression portions is 2 to 200 mm².

2. The absorbent article according to claim 1, wherein
an area rate of the high-compression portions in an arrangement region of the plurality of high-compression portions is 10 to 35%, and
a diameter of a largest inscribed circle inscribed in an outline of the non-high-compression portions is 30 mm or less.

3. The absorbent article according to claim 2, wherein
the high-compression portions have an outline with no inflection points or bending points, the diameter of the largest inscribed circle inscribed in the outline of the high-compression portions is 1 to 10 mm, and a circumferential length of the outline of the high-compression portions is 1 to 15 times a circumferential length of the largest inscribed circle inscribed in the outline of the high-compression portions.

4. The absorbent article according to claim 2 or 3, wherein
when a virtual grid formed by a first virtual straight line along a first direction repeatedly arranged at a first interval in a second direction inclined at 80 to 90° clockwise in a plan view with respect to the first virtual straight line and a second virtual straight line along the second direction repeatedly arranged at a second interval in the first direction is defined in the arrangement region of the plurality of high-compression portions, and smallest virtual quadrangles with intersections of the first virtual straight line and the second virtual straight line as vertices are defined,
the high-compression portions are constituted of first high-compression portions arranged at intersection positions of the first virtual straight line and the second virtual straight line, second high-compression portions respectively arranged between adjacent first high-compression portions on the first virtual straight line and between adjacent first high-compression portions on the second virtual straight line, and third high-compression portions arranged at intersection positions of diagonals of the virtual quadrangles,
the first high-compression portions have a circular shape centered on an intersection of the first virtual straight line and the second virtual straight line,
the second high-compression portions have an elliptical shape or a rounded rectangle shape with a center of gravity at a midpoint of each side and a major axis along each side of the virtual quadrangles, and
the third high-compression portions have an elliptical shape or a rounded rectangle shape with a center of gravity at the intersections of the diagonals and a major axis along a front-back direction of the virtual quadrangles or a circular shape centered on the intersections of the diagonals of the virtual quadrangles.

5. The absorbent article according to claim 4, wherein
the first high-compression portions have a diameter of 1 to 4 mm,
the second high-compression portions have a major axis length of 3 to 6 mm and a minor axis length equal to a diameter of the first high-compression portions,
the third high-compression portions have same dimensions and a same shape as the second high-compression portions with the exception of an orientation of the major axis being along the front-back direction, and
a minimum interval between adjacent first high-compression portions and second high-compression portions is 1 to 2 mm.

6. The absorbent article according to claim 4, wherein
the virtual grid has a diagonal grid shape of which the first virtual straight line is inclined at 40 to 50° clockwise in a plan view with respect to the front-back direction and the second virtual straight line is inclined at 40 to 50° counterclockwise in a plan view with respect to the front-back direction.

7. The absorbent article according to claim 1 or 2, wherein
a basis weight of the pulp fibers in the absorbent body is 100 to 500 g/m²,
a ratio by weight of pulp fibers to super-absorbent polymer particles in the absorbent body is 40:60 to 65:35,
a thickness of the non-high-compression portions is 3 to 13 mm, and
a thickness of the high-compression portions is 60 to 90% of the thickness of the non-high-compression portions.

8. The absorbent article according to claim 1 or 2, wherein
a percentage of particles of which a pre-swelling particle diameter is 500 µm or less in the super-absorbent polymer particles is 60% by mass or more, and
in all pulp fibers of the absorbent body, a percentage of pulp fibers with a fiber length of 0.5 mm or more and less than 1.1 mm is 40% or more by mass, and a percentage of pulp fibers with a fiber width of 10 µm or more and less than 35 µm is 90% or more by mass.

9. The absorbent article according to claim 8, wherein
the pulp fibers of the absorbent body are constituted of softwood pulp fibers and the hardwood pulp fibers, and
a mass ratio of the hardwood pulp fibers to the softwood pulp fibers is 25/75 or higher and 38/62 or lower.
